# EUROPEAN PATENT APPLICATION

(11) **EP 4 844 405 A1**
(43) Date of publication of application: **25.11.2026**
(21) Application number: 25741829.3
(22) Date of filing: 07.01.2025
(51) Int. Cl.: G01N 33/493, G01N 33/15, G01N 33/48, G01N 33/50, G01N 33/84, G01N 33/483

(54) **BIOMARKER FOR DETERMINING ONSET OF UROLITHIASIS AND USE THEREOF**

(30) Priority: 17.01.2024 JP 2024004939; 06.08.2024 JP 2024129618
(71) Applicant: The University of Osaka, Suita-shi, Osaka 565-0871 (JP); PUBLIC UNIVERSITY CORPORATION NAGOYA CITY UNIVERSITY, Nagoya-shi, Aichi 467-8601 (JP); UHA Mikakuto Co., Ltd., Yamatokooriyama-shi, Nara 639-1031 (JP)
(72) Inventor: HIRAO MARUYAMA, Mihoko, Suita-shi, Osaka 565-0871 (JP); TANAKA, Yutaro, Suita-shi, Osaka 565-0871 (JP); MORI, Yusuke, Suita-shi, Osaka 565-0871 (JP); YOSHIMURA, Masashi, Suita-shi, Osaka 565-0871 (JP); YASUI, Takahiro, Nagoya-shi, Aichi 467-8601 (JP); OKADA, Atsushi, Nagoya-shi, Aichi 467-8601 (JP); YAMADA, Yasumasa, Yamatokooriyama-shi, Nara 639-1031 (JP); MATSUKAWA, Taiji, Yamatokooriyama-shi, Nara 639-1031 (JP)
(74) Representative: Pelster Behrends Patentanwälte PartG mbB
(86) International application number: PCT/JP2025/000230
(87) International publication number: WO 2025/154584

(57) **Abstract**

Provided is a new biomarker for determining development of urolithiasis. A biomarker for determining development of urolithiasis of the present disclosure is characterized by including at least one of calcium phosphate crystals or amorphous calcium phosphate in urine.

## Description

### TECHNICAL FIELD

The present disclosure relates to a biomarker for determining development of urolithiasis and use thereof.

### BACKGROUND ART

Urolithiasis is a disease that involves formation of a calculus in a urinary tract, where the formed calculus is called a urinary calculus. It is known that calcium oxalate calculi account for the majority of urinary calculi. For this reason, development of urolithiasis has been determined based on a daily excretion amount of an inorganic component such as oxalic acid, calculated by a 24-hour urine collection test.

### Citation List

### Non-Patent Literature

Non-patent literature 1: Saeed R. Khan et al, Kidney stones, Nat Rev Dis Primers, (2016) 25:2:16008.
Non-patent literature 2: I.A. Karampas and C.G. Kontoyannis, Characterization of calcium phosphates mixtures, Vibrational Spectroscopy, 64 (2013) 126-133.
Non-patent literature 3: Liga Berzina-Cimdina and Natalija Borodajenko, Research of Calcium Phosphates Using Fourier Transform Infrared Spectroscopy, Infrared Spectroscopy - Materials Science, Engineering and Technology, (2012) 123-148.

### SUMMARY OF INVENTION

### Technical Problem

However, in recent years, it has been reported that organic components such as proteins play a promotive or suppressive role in a growth process of a urinary calculus (Non-Patent Literature 1). For this reason, a conventional method for evaluating only inorganic components is incapable of grasping formation of a urinary calculus under a comprehensive environment including organic components, resulting in failure in determining development of urolithiasis with high accuracy.

Hence, it is an object of the present disclosure to provide a new biomarker for determining development of urolithiasis, capable of determining development of urolithiasis with high accuracy.

### Solution to Problem

To achieve the above object, the present disclosure provides a biomarker for determining development of urolithiasis, including at least one of calcium phosphate crystals or amorphous calcium phosphate in urine.

The present disclosure also provides a method for determining development of urolithiasis, including detecting the biomarker of the present disclosure in urine from a subject, and determining development of urolithiasis in the subject based on a result of the detection of the biomarker.

The present disclosure also provides a method for screening for a candidate substance for suppressing urolithiasis, including selecting a formation-inhibiting substance that inhibits formation of at least one of calcium phosphate crystals or amorphous calcium phosphate from test substances, as the candidate substance for suppressing urolithiasis.

### Advantageous Effects of Invention

The present disclosure can achieve determination of development of urolithiasis with high accuracy.

### BRIEF DESCRIPTION OF DRAWINGS

[FIG. 1] FIG. 1 shows photographs of observation images of an insolubilized material of calcium oxalate formed under temperature conditions of 20°C, 10°C, and 4°C in Example 1 of the present disclosure, obtained by an optical microscope.
[FIG. 2] FIG. 2 is a table showing basic data of patients with urolithiasis in Example 1 of the present disclosure.
[FIG. 3] FIG. 3 shows photographs of results of detection of an insolubilized material of calcium phosphate by an optical microscope in Example 1 of the present disclosure.
[FIG. 4] FIG. 4 shows graphs of detection results of an insolubilized material of calcium phosphate by Raman spectroscopic analysis in Example 1 of the present disclosure. [FIG. 5] FIG. 5 is a table showing a result of a statistical test between two groups of a group in which an insolubilized material was not observed and a group in which an insolubilized material of calcium oxalate was observed in Example 2 of the present disclosure.
[FIG. 6] FIG. 6 is a table showing a result of a statistical test between two groups of a group in which an insolubilized material was not observed and a group in which an insolubilized material of calcium phosphate was observed in Example 2 of the present disclosure.
[FIG. 7] FIG. 7 is a table showing a result of a statistical test between two groups of a group in which an insolubilized material of calcium oxalate was observed and a group in which an insolubilized material of calcium phosphate was observed in Example 2 of the present disclosure.
[FIG. 8] FIG. 8 is a photograph showing an observation image of a thin slice of a calcium oxalate calculus by a polarizing microscope under an open-nicol condition in Example 3 of the present disclosure.
[FIG. 9] FIG. 9 shows photographs of mapping results of four elements in Area A on a thin slice of a calcium oxalate calculus by fluorescent X-ray analysis in Example 3 of the present disclosure.
[FIG. 10] FIG. 10 is a photograph showing an observation image of Area A on a thin slice of a calcium oxalate calculus by a polarizing microscope under a cross-nicol condition in Example 3 of the present disclosure.
[FIG. 11] FIG. 11 is a graph showing a result of detection of a calcium phosphate phase of Area A on a thin slice of a calcium oxalate calculus by a laser Raman microscope in Example 3 of the present disclosure.
[FIG. 12] FIG. 12 is a photograph showing an observation image of immunofluorescence staining of Area A on a thin slice of a calcium oxalate calculus by a fluorescence microscope in Example 3 of the present disclosure.
[FIG. 13] FIG. 13 shows photographs of mapping results of four elements in Area B on a thin slice of a calcium oxalate calculus by fluorescent X-ray analysis in Example 3 of the present disclosure.
[FIG. 14] FIG. 14 is a photograph showing an observation image of Area B on a thin slice of a calcium oxalate calculus by a polarizing microscope under a cross-nicol condition in Example 3 of the present disclosure.
[FIG. 15] FIG. 15 is a graph showing a result of detection of a calcium phosphate phase of Area B on a thin slice of a calcium oxalate calculus by a laser Raman microscope in Example 3 of the present disclosure.
[FIG. 16] FIG. 16 shows photographs of observation images of immunofluorescence staining of Area B on a thin slice of a calcium oxalate calculus by a fluorescence microscope, and a photograph showing an observation image of an insolubilized material of calcium phosphate in a urine sample by an optical microscope in Example 3 of the present disclosure.
[FIG. 17] FIG. 17 is a table showing basic data of a group without recurrence of urolithiasis and a group with recurrence of urolithiasis in Example 4 of the present disclosure.
[FIG. 18] FIG. 18 is a table showing a result of a statistical test between two groups of a group without recurrence of urolithiasis and a group with recurrence of urolithiasis in Example 4 of the present disclosure.
[FIG. 19] FIG. 19 is a table showing a result of logistic regression analysis in Example 4 of the present disclosure.
[FIG. 20] FIG. 20 is a table showing basic data of subjects in Example 5 of the present disclosure.
[FIG. 21] FIG. 21 is a table showing a result of a statistical test for a 24-hour urine collection test result in Example 5 of the present disclosure.
[FIG. 22] FIG. 22 shows graphs of results of a statistical test for a 24-hour urine collection test result in Example 5 of the present disclosure.
[FIG. 23] FIG. 23 shows photographs of results of detection of each insolubilized material in a cooled urine sample by an optical microscope in Example 5 of the present disclosure.
[FIG. 24] FIG. 24 is a table showing a result of a statistical test between respective groups for the number of subjects, in whom each insolubilized material was observed in their cooled urine samples, in Example 5 of the present disclosure.
[FIG. 25] FIG. 25 is a graph showing a result of a statistical test between respective groups for the number of subjects, in whom an insolubilized material of Brushite was observed in their cooled urine samples, in Example 5 of the present disclosure.
[FIG. 26] FIG. 26 is a table showing a result of logistic regression analysis in Example 5 of the present disclosure.
[FIG. 27] FIG. 27 shows graphs of correlation between SI.COD and SI.Brushite in subjects, in whom an insolubilized material was observed in their cooled urine samples, in Example 5 of the present disclosure.
[FIG. 28] FIG. 28 shows photographs comparing results of detection of an insolubilized material of Brushite in cooled urine samples by an optical microscope among three patients: a patient with early recurrence of urolithiasis, a patient with late recurrence of urolithiasis, and a patient with no recurrence of urolithiasis, in Example 5 of the present disclosure.
[FIG. 29] FIG. 29 is a graph showing a correlation between SI.COD and SI.Brushite of each group of patients with urolithiasis in Example 5 of the present disclosure.
[FIG. 30] FIG. 30 is a graph showing a result of detection of an insolubilized material of calcium phosphate by Raman spectroscopic analysis in Example 5 of the present disclosure.

### DESCRIPTION OF EMBODIMENTS

### Definition

As used herein, "urolithiasis" refers to a disease that involves formation of a calculus in a urinary tract. The calculus formed in a urinary tract is referred to as, for example, a urinary calculus (hereinafter, also referred to as a calculus). The urinary tract is, for example, a kidney, a ureter, a bladder, or a urethra. Examples of the kidney include a right kidney, a left kidney, and both kidneys. Examples of the ureter include a right ureter, a left ureter, and both ureters. The urolithiasis is diagnosed, for example, by recognizing presence of the urinary calculus by an imaging examination. The imaging examination includes, for example, a plain CT examination, intravenous urography (IVU), a plain X-ray examination, ultrasonography, and the like. Examples of the plain X-ray examination include plain radiography of kidney, ureter, and bladder.

As described above, the urinary calculus is not particularly limited, and is, for example, a calculus formed in the urinary tract. Examples of components of the urinary calculus include cystine calculi, uric acid calculi, magnesium ammonium phosphate calculi, calcium oxalate calculi, calcium phosphate calculi, and mixed calculi thereof. The components of the urinary calculus may be, for example, calcium oxalate calculi, calcium phosphate calculi, or mixed calculi of calcium oxalate calculi and calcium phosphate calculi. The components of the urinary calculus can be identified, for example, by calculus analysis. The calculus analysis is performed, for example, by infrared spectroscopy or the like. A site where the urinary calculus is present is, for example, the urinary tract, and is a kidney, a ureter, a bladder, and a urethra. Examples of the kidney include a right kidney, a left kidney, and both kidneys. Examples of the ureter include a right ureter, a left ureter, and both ureters. The site where the urinary calculus is present may be, for example, both kidneys or both ureters. The site where the urinary calculus is present can be specified, for example, by the imaging examination.

As used herein, "calcium phosphate" refers to a generic term for inorganic salts containing a calcium ion and a phosphate ion. Examples of the calcium phosphate include monobasic calcium phosphate (calcium dihydrogen phosphate), dibasic calcium phosphate (also referred to as calcium hydrogen phosphate or calcium monohydrogen phosphate), tribasic calcium phosphate (tricalcium phosphate), calcium diphosphate (calcium pyrophosphate), calcium hydroxyphosphate (also referred to as hydroxyapatite), carbonate apatite (also referred to as carbonate-containing hydroxyapatite or carbonate apatite), tetracalcium phosphate, octacalcium phosphate, and amorphous calcium phosphate. Examples of the tribasic calcium phosphate (the tricalcium phosphate) include α-tricalcium phosphate and β-tricalcium phosphate. The phosphate ion is, for example, a diphosphate ion when the calcium phosphate is the calcium diphosphate (the calcium pyrophosphate). The amorphous calcium phosphate is also referred to as, for example, an amorphous form of calcium phosphate. The calcium phosphate may be, for example, a hydrate of the calcium phosphate, or may be an anhydrate of the calcium phosphate. Further, the calcium phosphate may be, for example, calcium phosphate mainly contained in the urinary calculus or a urine sample of a patient with urolithiasis, and examples thereof include hydroxyapatite, calcium hydrogen phosphate, carbonate apatite, tricalcium phosphate, and the like.

As used herein, "crystal" refers to a solid in which atoms, molecules, or ions are periodically arranged in three-dimensional directions. The periodicity of the crystal has, for example, long-range order. The crystal is classified into, for example, a covalent crystal, an ionic crystal, a metallic crystal, a molecular crystal, and the like, based on the type of chemical bond. Examples of the crystal include a single crystal and a polycrystal. The crystal includes, for example, any formed material in a formation process of the crystal, such as a crystal nucleus. In the present specification, the crystal may include, for example, a quasicrystal. The quasicrystal can be said to be, for example, a crystal having no translational symmetry. The crystal can be formed, for example, by cooling, evaporation, or the like. The crystal is also referred to as, for example, a crystallized material, an insolubilized material, a solid-phase material, a precipitated material, a solution-crystallized material, a separated material, or the like. Formation of the crystal is also referred to as, for example, crystallization, insolubilization, formation of a solid-phase, precipitation, crystallization of a solution, separation, or the like. A process of forming the crystal is also referred to as, for example, crystallizing, insolubilizing, forming a solid-phase, precipitating, crystallizing a solution, separating, or the like. Processing for forming the crystal is also referred to as, for example, crystallization processing, insolubilization processing, solid-phase formation processing, precipitation processing, solution-crystallization processing, separation processing, or the like.

As used herein, "amorphous" refers to being a solid in which atoms, molecules, or ions are not periodically arranged in three-dimensional directions. The periodicity of the amorphous form has, for example, short-range order, but does not have long-range order. The amorphous is also referred to as, for example, non-crystalline. A material with the amorphous form includes, for example, any formed material in a formation process of the amorphous material. The amorphous material can be formed, for example, by cooling, evaporation, or the like. The amorphous material is also referred to as, for example, an amorphized material, an insolubilized material, a solid-phase material, a precipitated material, a solution-crystallized material, a separated material, or the like. Formation of the amorphous material is also referred to as, for example, amorphization, insolubilization, formation of a solid-phase, precipitation, crystallization of a solution, separation, or the like. A process of forming the amorphous material is also referred to as, for example, amorphizing, insolubilizing, forming a solid-phase, precipitating, crystallizing a solution, separating, or the like. Processing of forming the amorphous material is also referred to as, for example, amorphization processing, insolubilization processing, solid-phase formation processing, precipitation processing, solution-crystallization processing, separation processing, or the like.

Hereinafter, embodiments and examples of the present disclosure will be described with reference to the drawings. The present disclosure is not limited to the following embodiments and examples. Further, the descriptions of the respective embodiments can incorporate each other unless otherwise specified, and the configurations of the respective embodiments can be combined unless otherwise specified.

### Biomarker for determining development of urolithiasis

A biomarker for determining development of urolithiasis according to the present disclosure (hereinafter, also referred to as the biomarker of the present disclosure) is not particularly limited, and is, for example, a biomarker including at least one of calcium phosphate crystals or amorphous calcium phosphate in urine. The biomarker of the present disclosure is characterized in that at least one of calcium phosphate crystals or amorphous calcium phosphate is used as a biomarker for determining development of urolithiasis, and other configurations and conditions are not particularly limited. The biomarker of the present disclosure allows determination of development of urolithiasis in the subject, for example, by detection of the biomarker of the present disclosure in urine from a subject. The biomarker of the present disclosure can be used in a method for determining development of urolithiasis according to the present disclosure, and a method for screening for a candidate substance for suppressing urolithiasis according to the present disclosure, which will be described later.

As a result of intensive studies, the inventors found that at least one of calcium phosphate crystals or amorphous calcium phosphate in urine is associated with development of urolithiasis, and established the present invention. Therefore, detection of the biomarker of the present disclosure allows development of urolithiasis in a subject to be determined. Further, a candidate substance for suppressing urolithiasis can also be obtained by screening using the biomarker of the present disclosure.

The biomarker of the present disclosure may be, for example, a biomarker for determining recurrence of urolithiasis. The recurrence of urolithiasis is, for example, reformation of a urinary calculus (newly nucleated, grown, aggregated, and solidified) in a patient in whom no urinary calculus was detected by an imaging examination or the like after treatment of urolithiasis. For this reason, the recurrence of urolithiasis does not include, for example, a case where a urinary calculus is regrown in a patient in whom a residual calculus of a urinary calculus was detected by an imaging examination or the like after treatment of urolithiasis. The recurrence of urolithiasis may be, for example, early recurrence of urolithiasis or late recurrence of urolithiasis. The early recurrence of urolithiasis is, for example, reformation of a urinary calculus within 1 year, within 2 years, within 3 years, within 4 years, or within 5 years from a point when no urinary calculus was detected by an imaging examination or the like after treatment of urolithiasis. The late recurrence of urolithiasis is, for example, reformation of a urinary calculus after more than 5 years, more than 6 years, more than 7 years, more than 8 years, more than 9 years, or more than 10 years from a point when no urinary calculus was detected by an imaging examination or the like after treatment of urolithiasis. A site where the urinary calculus is reformed may be, for example, the same site as a site where a urinary calculus was present before treatment of urolithiasis, or may be a site different from a site where a urinary calculus was present before treatment of urolithiasis. The treatment includes, for example, spontaneous calculus passage, a treatment for promoting spontaneous calculus passage, a treatment by calculus crushing, and the like. The spontaneous calculus passage is, for example, that a urinary calculus is spontaneously excreted without depending on the treatment for promoting spontaneous calculus passage, the treatment by calculus crushing, or the like. Examples of the treatment for promoting spontaneous calculus passage include a large intake of water, body movement, oral medicine, and the like. Examples of the treatment by calculus crushing include extracorporeal shock wave lithotripsy, endoscopic lithotripsy, percutaneous lithotripsy, abdominal surgery, and the like. For the imaging examination, for example, the aforementioned description can be applied.

In the present disclosure, an origin of at least one of calcium phosphate crystals or amorphous calcium phosphate is not particularly limited, and can be appropriately set depending on, for example, a type of a subject. Examples of the origin include, for example, a human, a non-human animal excluding a human, and the like. Examples of the non-human animal excluding a human include a mouse, a rat, a dog, a cat, a monkey, a rabbit, a sheep, and a horse. In the present disclosure, at least one of the calcium phosphate crystals or the amorphous calcium phosphate is preferably, for example, derived from a human.

The biomarker of the present disclosure may be used alone, or may be used in combination with a biomarker for determining development of urolithiasis other than the biomarker of the present disclosure (hereinafter, also referred to as the other biomarker). The other biomarker can be detected, for example, by a urine test, a blood test, calculus analysis, an imaging examination, or the like. The urine test includes, for example, a 24-hour urine collection test, a urine dipstick test, a urine sediment test, and the like. Examples of the other biomarker detected by the 24-hour urine collection test include sodium, potassium, chlorine, calcium, inorganic phosphorus, magnesium, creatinine, uric acid, urea nitrogen, oxalic acid, citric acid, and an amino acid. Examples of the other biomarker detected by the urine dipstick test include pH, an occult blood reaction, a white blood cell count, nitrite, and turbidity. Examples of the other biomarker detected by the urine sediment test include a red blood cell count, a white blood cell count, salts, and crystals. Examples of the other biomarker detected by the blood test include kidney function test items (creatinine, urea nitrogen, and the like), uric acid, calcium, inorganic phosphorus, and CRP. Examples of the other biomarker detected by the calculus analysis include a cystine calculus, a uric acid calculus, a magnesium ammonium phosphate calculus, a calcium oxalate calculus, a calcium phosphate calculus, or a mixed calculus thereof, and the like. For the imaging examination, for example, the aforementioned description can be applied.

Examples of the type of the urine include early morning urine, random urine, and accumulated urine. The early morning urine is, for example, first urine immediately after waking up, and is also referred to as early morning first urine. The random urine is, for example, urine collected at any time, and is also referred to as spot urine. The accumulated urine is, for example, urine obtained by collecting all urine excreted in a certain period of time, and examples thereof include 24-hour accumulated urine. A collection condition of the accumulated urine may be, for example, acidic accumulated urine or normal accumulated urine. The acidic accumulated urine is, for example, accumulated urine containing an acidic substance. Examples of the acidic substance include hydrochloric acid. A normality of the hydrochloric acid may be, for example, 1 to 6 N. The normality of the hydrochloric acid is preferably, for example, 6 N. The normal accumulated urine is, for example, accumulated urine not containing an acidic substance. The accumulated urine may contain, for example, a stabilizer, a preservative, and the like. In addition, a method for collecting the urine may be, for example, spontaneous excretion, excretion by a urinary catheter, or excretion by suprapubic needle aspiration. A collection amount of the urine may be, for example, whole urine or partial urine. The whole urine is, for example, one obtained by collecting the whole amount of excreted urine. The partial urine is, for example, one obtained by collecting a part of excreted urine, and examples thereof include first urine, midstream urine, and last urine. The first urine is, for example, urine collected immediately after the start of urination. The last urine is, for example, urine collected near the end of urination. The midstream urine is, for example, urine collected by avoiding the first urine and the last urine. The urine may be, for example, aliquoted urine subdivided from a collection container.

The biomarker of the present disclosure can be used as a marker for development of urolithiasis, and specifically, for example, is preferably used as a marker for recurrence of urolithiasis.

The biomarker of the present disclosure can be detected, for example, by a microscopic examination method, an automatic analysis method, Raman spectroscopy, infrared spectroscopy, terahertz spectroscopy, and the like, which will be described later.

The biomarker of the present disclosure can be used in a determination method of the present disclosure, a suppressing substance and suppressing method of the present disclosure, and a screening method of the present disclosure, which will be described later.

### Method for determining development of urolithiasis

The method for determining development of urolithiasis according to the present disclosure (hereinafter, also referred to as the determination method of the present disclosure) is not particularly limited, and is, for example, a determination method including detecting the biomarker of the present disclosure in urine from a subject; and determining development of urolithiasis in the subject based on a result of the detection of the biomarker. The determination method of the present disclosure is characterized in that, in the detecting, the biomarker of the present disclosure in the urine from the subject is detected, and other processes and conditions are not particularly limited. The determination method of the present disclosure can also be referred to as, for example, an evaluation method, an examination method, or the like. The present disclosure allows development of urolithiasis to be determined. For the determination method of the present disclosure, the description of the biomarker of the present disclosure can be applied.

By the determination method of the present disclosure, for example, the possibility of development of urolithiasis, presence or absence of development of urolithiasis (whether or not urinary calculus is formed), a prognosis state, and the like can be evaluated. As described above, the target urolithiasis is preferably recurrence of urolithiasis. Further, the target urolithiasis may be initial urolithiasis, or may be urolithiasis that has recurred twice or more.

The determination method of the present disclosure may be, for example, a determination method that includes the determining in which recurrence of urolithiasis in the subject is determined. By the determination method of the present disclosure, for example, the possibility of recurrence of urolithiasis, presence or absence of recurrence of urolithiasis (whether or not urinary calculus is formed), a prognosis state, and the like can be evaluated. For the recurrence of urolithiasis, for example, the above description can be applied.

In the determination method of the present disclosure, the subject includes, for example, a human, a non-human animal excluding a human, and the like. The non-human animal excluding a human includes, for example, a mouse, a rat, a dog, a cat, a monkey, a rabbit, a sheep, a horse, and the like.

In the determination method of the present disclosure, examples of the type of the urine include early morning urine, random urine, and accumulated urine. The early morning urine is, for example, first urine immediately after waking up, and is also referred to as early morning first urine. The random urine is, for example, urine collected at any time, and is also referred to as spot urine. The accumulated urine is, for example, urine obtained by collecting all urine excreted in a certain period of time, and examples thereof include 24-hour accumulated urine. A collection condition of the accumulated urine may be, for example, acidic accumulated urine or normal accumulated urine. The acidic accumulated urine is, for example, accumulated urine containing an acidic substance. Examples of the acidic substance include hydrochloric acid. A normality of the hydrochloric acid may be, for example, 1 to 6 N. The normality of the hydrochloric acid is preferably, for example, 6 N. The normal accumulated urine is, for example, accumulated urine not containing an acidic substance. The accumulated urine may contain, for example, a stabilizer, a preservative, and the like. In addition, a method for collecting the urine may be, for example, spontaneous excretion, excretion by a urinary catheter, or excretion by suprapubic needle aspiration. A collection amount of the urine may be, for example, whole urine or partial urine. The whole urine is, for example, one obtained by collecting the whole amount of excreted urine. The partial urine is, for example, one obtained by collecting a part of excreted urine, and examples thereof include first urine, midstream urine, and last urine. The first urine is, for example, urine collected immediately after the start of urination. The last urine is, for example, urine collected near the end of urination. The midstream urine is, for example, urine collected by avoiding the first urine and the last urine. The urine may be, for example, aliquoted urine subdivided from a collection container.

The determination method of the present disclosure may be, for example, a determination method including performing insolubilization processing on the urine from the subject to form the biomarker of the present disclosure. In the performing insolubilization processing, the insolubilization processing is not particularly limited, and for example, may employ a known insolubilization method. The insolubilization processing can also be referred to as, for example, crystallization processing, amorphization processing, solid-phase formation processing, precipitation processing, solution-crystallization processing, separation processing, or the like. The performing insolubilization processing can also be referred to as, for example, crystallizing, amorphizing, forming a solid-phase, precipitating, crystallizing a solution, separating, or the like. The insolubilization processing can be performed by, for example, addition of an inorganic component, addition of a poor solvent, addition of a seed crystal, addition of a pH adjuster, addition of an ionic strength adjuster, or the like. Further, the insolubilization processing can also be performed by, for example, supersaturation described later. The addition of an inorganic component is, for example, adding a solution containing sodium oxalate and calcium chloride to the urine from the subject. Further, the addition of an inorganic component may be, for example, adding a solution containing calcium phosphate, a solution containing inorganic phosphorus, a solution containing calcium, or the like to the urine from the subject. An addition rate of the inorganic component may be, for example, low or high. An addition rate of the poor solvent may be, for example, low or high. The addition of the pH adjuster is, for example, adding an alkaline agent to the urine from the subject. Examples of the alkaline agent include sodium hydroxide and calcium hydroxide.

The determination method of the present disclosure may be, for example, a determination method that includes the performing insolubilization processing in which the insolubilization processing is supersaturation. Supersaturation is, for example, a state in which a solute is contained in a solution in an amount equal to or greater than a solubility. In the case of the present disclosure, the supersaturation is, for example, a state in which calcium phosphate is contained in the urine from the subject in an amount equal to or greater than a solubility. The solubility is, for example, a limit amount of a solute that can be dissolved in a certain amount of a solvent. The supersaturation may be, for example, a state in which a solute is contained in a solution in an amount equal to or greater than a supersolubility. The supersaturation is, for example, changing a solution to the state of supersaturation. In the case of the present disclosure, the supersaturation is, for example, changing the urine from the subject to the state of supersaturation. In the case of the present disclosure, the supersaturation can also be referred to as, for example, supersaturation of calcium phosphate in the urine from the subject. For the supersaturation, for example, an insolubilization processing by the addition of an inorganic component, the addition of a poor solvent, the addition of a seed crystal, the addition of a pH adjuster, the addition of an ionic strength adjuster, and the like may be performed in parallel, or may be performed before or after the supersaturation. The supersaturation can be caused by, for example, cooling the urine from the subject, or evaporating the urine from the subject. The evaporation includes, for example, evaporation by heating, evaporation by reduced pressure, and the like. Since the patients with urolithiasis have, for example, calcium phosphate in their urine, it is presumed that crystal nuclei of the biomarker of the present disclosure are generated by the supersaturation, whereby the biomarker of the present disclosure is formed. Further, since the patients with urolithiasis have, for example, crystal nuclei of the biomarker of the present disclosure in their urine, it is presumed that the crystal nuclei grow by the supersaturation, whereby the biomarker of the present disclosure is formed. However, a formation mechanism of the biomarker of the present disclosure by the supersaturation is by no means limited by, for example, each of the above presumptions. The supersaturation allows, for example, calcium phosphate in the urine from the subject to be supersaturated, whereby the biomarker of the present disclosure can be formed. For the calcium phosphate, for example, the above description can be applied and the calcium phosphate includes any formed material in a formation process of the biomarker of the present disclosure.

The supersaturation may be, for example, cooling. For the supersaturation by the cooling, it is presumed that, for example, calcium phosphate in the urine from the subject is changed to a state of supersaturation by the cooling, crystal nuclei of the biomarker of the present disclosure are further generated by the cooling, and the crystal nuclei grow, whereby the biomarker of the present disclosure is formed. Further, for the supersaturation by the cooling, it is presumed that, for example, the crystal nuclei of the biomarker of the present disclosure contained in the urine from the subject are grown by the cooling, whereby the biomarker of the present disclosure is formed. However, the supersaturation by the cooling is by no means limited by, for example, each of the above presumptions. Examples of an apparatus used for the cooling include a cold storage, a refrigerator, a refrigerator-freezer, a low-temperature incubator, a low-temperature thermostatic chamber, and a cold and warm storage. The apparatus used for the cooling may be, for example, for household use or for pharmaceutical use. A temperature of the cooling may be, for example, 0 to 20°C, 2 to 18°C, 4 to 16°C, 6 to 14°C, 8 to 12°C, or the like. The temperature of the cooling may be, for example, 0 to 10°C, 2 to 8°C, 4 to 6°C, or the like. The temperature of the cooling is preferably, for example, 0 to 4°C. Further, the temperature of the cooling may be, for example, constant, or may be different in the early stage of the cooling, the middle stage of the cooling, and the late stage of the cooling. A rate of the cooling may be, for example, constant, or may be different in the early stage of the cooling, the middle stage of the cooling, and the late stage of the cooling. A time of the cooling is not particularly limited, and examples thereof include 24 hours or more, 36 hours or more, and 48 hours or more. The urine during the cooling may be, for example, left to stand or stirred. The stirring can be performed by, for example, a stirrer or the like. A speed of the stirring may be, for example, constant, or may be different in the early stage of the cooling, the middle stage of the cooling, and the late stage of the cooling. For the supersaturation by the cooling, for example, the insolubilization processing by the addition of an inorganic component, the addition of a poor solvent, the addition of a seed crystal, the addition of a pH adjuster, the addition of an ionic strength adjuster, and the like may be performed in parallel, or may be performed before or after the cooling.

The determination method of the present disclosure may be, for example, a determination method in which when the biomarker of the present disclosure in the urine from the subject is detected in the detecting, it is determined that the subject has developed urolithiasis in the determining. The detection of the biomarker of the present disclosure in the detecting may be, for example, analysis of the presence or absence of the biomarker of the present disclosure in the urine (qualitative analysis) or analysis of an amount of the biomarker of the present disclosure in the urine (quantitative analysis).

A detection target in the detecting is the biomarker of the present disclosure, and specifically includes at least one of calcium phosphate crystals or amorphous calcium phosphate.

In the detecting, the biomarker of the present disclosure may be used alone, or may be used in combination with a biomarker for determining development of urolithiasis other than the biomarker of the present disclosure (the other biomarker). For the other biomarker, for example, the above description can be applied.

In the detecting, a method for detecting the biomarker of the present disclosure is not particularly limited, and may be a known method. Specific examples of the method for detecting the biomarker of the present disclosure include a microscopic examination method, an automated analysis method, Raman spectroscopy, infrared spectroscopy, an X-ray diffraction method, and terahertz spectroscopy.

The microscopic examination method can be performed using, for example, an optical microscope, a polarizing microscope, an electron microscope, or the like. In the microscopic examination method using the optical microscope, it is known that the biomarker of the present disclosure can be observed, for example, as rosette-like or plate-like, colorless to grayish white crystals. Further, it is known that since the biomarker of the present disclosure dissolves and disappears in, for example, acetic acid, hydrochloric acid, or the like, the biomarker of the present disclosure can be differentiated from other physical components based on solubility with an acid. Therefore, the biomarker of the present disclosure can be differentiated from other physical components based on, for example, a shape, a color tone, a size, solubility with an acid, or the like of the biomarker of the present disclosure. Further, for the biomarker of the present disclosure, for example, presence or absence of the biomarker of the present disclosure can be qualitatively analyzed, or an amount of the biomarker of the present disclosure can be quantitatively analyzed, based on a result of the differentiation. In this case, the amount of the biomarker of the present disclosure may be, for example, the number, the size, or the like of the biomarker of the present disclosure.

The automated analysis method can be performed by, for example, an image processing method, a cytometry method, or the like. The automated analysis method can also be referred to as, for example, a urinary physical components analysis method. The image processing method is, for example, a method with automation of the microscopic examination method, where image analysis on a captured image by a CCD camera or the like allows presence or absence of the biomarker of the present disclosure to be qualitatively analyzed, or an amount of the biomarker of the present disclosure to be quantitatively analyzed. In the cytometry method, for example, analysis of scattered light in response to laser light irradiation allows presence or absence of the biomarker of the present disclosure to be qualitatively analyzed, or an amount of the biomarker of the present disclosure to be quantitatively analyzed.

In the Raman spectroscopy, when at least one of calcium hydrogen phosphate crystals or amorphous calcium hydrogen phosphate contained in the biomarker of the present disclosure is detected, it is known that the biomarker of the present disclosure shows specific peaks at Raman shifts of around 990 cm⁻¹ and 875 to 880 cm⁻¹, for example (Non-Patent Literature 2). Therefore, the biomarker of the present disclosure can be differentiated from other contained components based on, for example, the specific peaks at the Raman shifts of around 990 cm⁻¹ and 875 to 880 cm⁻¹ of the biomarker of the present disclosure, or the like. Further, for the biomarker of the present disclosure, for example, presence or absence of the biomarker of the present disclosure can be qualitatively analyzed, or an amount of the biomarker of the present disclosure can be quantitatively analyzed, based on a result of the differentiation. In this case, the amount of the biomarker of the present disclosure may be, for example, a concentration calculated by applying a Raman scattering intensity of the specific peaks at the Raman shifts of around 990 cm⁻¹ and 875 to 880 cm⁻¹ of the biomarker of the present disclosure to a calibration model prepared from a known amount of a standard sample. Further, Non-Patent Literature 2 describes that at least one of hydroxyapatite crystals or amorphous hydroxyapatite, at least one of carbonate apatite crystals or amorphous carbonate apatite, and at least one of tricalcium phosphate crystals or amorphous tricalcium phosphate, which are contained in the biomarker of the present disclosure, show a specific peak at a specific Raman shift in the Raman spectroscopy. Therefore, each of the biomarkers of the present disclosure can be differentiated from other contained components based on, for example, the specific peak at the Raman shift described in Non-Patent Literature 2. Further, for each of the biomarkers of the present disclosure, for example, presence or absence of each of the biomarkers of the present disclosure can be qualitatively analyzed, or an amount of each of the biomarkers of the present disclosure can be quantitatively analyzed, based on a result of the differentiation. Note that, the description in Non-Patent Literature 2 is an exemplification, and as the Raman shift of the biomarker of the present disclosure in the Raman spectroscopy, for example, a known one can be used, and is by no means limited by the description in Non-Patent Literature 2.

Examples of the infrared spectroscopy include dispersive infrared spectroscopy, and Fourier transform infrared spectroscopy (FTIR). Non-Patent Literatures 2 and 3 describe that at least one of calcium hydrogen phosphate crystals or amorphous calcium hydrogen phosphate, at least one of hydroxyapatite crystals or amorphous hydroxyapatite, at least one of carbonate apatite crystals or amorphous carbonate apatite, and at least one of tricalcium phosphate crystals or amorphous tricalcium phosphate, which are contained in the biomarker of the present disclosure, show a specific peak at a specific wavenumber in the FTIR method. Therefore, the biomarker of the present disclosure can be differentiated from other contained components based on, for example, the specific peak at the wavenumber described in Non-Patent Literatures 2 and 3. Further, for the biomarker of the present disclosure, for example, presence or absence of the biomarker of the present disclosure can be qualitatively analyzed, or an amount of the biomarker of the present disclosure can be quantitatively analyzed, based on a result of the differentiation. Note that, the descriptions in Non-Patent Literatures 2 and 3 are exemplifications, and as the wavenumber for the biomarker of the present disclosure in the infrared spectroscopy, for example, a known one can be used, and is by no means limited by the descriptions in Non-Patent Literatures 2 and 3.

Examples of the X-ray diffraction method include a single crystal X-ray diffraction method, and a powder X-ray diffraction (PXRD) method. Non-Patent Literature 2 describes that at least one of calcium hydrogen phosphate crystals or amorphous calcium hydrogen phosphate, at least one of hydroxyapatite crystals or amorphous hydroxyapatite, at least one of carbonate apatite crystals or amorphous carbonate apatite, and at least one of tricalcium phosphate crystals or amorphous tricalcium phosphate, which are contained in the biomarker of the present disclosure, show a specific peak at a specific diffraction angle 2*θ* in the PXRD method. Therefore, the biomarker of the present disclosure can be differentiated from other contained components based on, for example, the specific peak at the diffraction angle 2*θ* described in Non-Patent Literature 2. Further, for the biomarker of the present disclosure, for example, presence or absence of the biomarker of the present disclosure can be qualitatively analyzed, or an amount of the biomarker of the present disclosure can be quantitatively analyzed, based on a result of the differentiation. Note that, the description in Non-Patent Literature 2 is an exemplification, and as the diffraction angle 2*θ* of the biomarker of the present disclosure in the X-ray diffraction method, for example, a known one can be used, and is by no means limited by the description in Non-Patent Literature 2.

Examples of the terahertz spectroscopy include, for example, Fourier transform infrared spectroscopy (FTIR), and terahertz time-domain spectroscopy (THz-TDS). In the terahertz spectroscopy, the biomarker of the present disclosure shows, for example, a specific peak at a specific terahertz frequency. Therefore, the biomarker of the present disclosure can be differentiated from other contained components based on the specific peak at the specific terahertz frequency by utilizing, for example, a known specific terahertz frequency of the biomarker of the present disclosure. Further, for the biomarker of the present disclosure, for example, presence or absence of the biomarker of the present disclosure can be qualitatively analyzed, or an amount of the biomarker of the present disclosure can be quantitatively analyzed, based on a result of the differentiation.

In the determination method of the present disclosure, for example, in the detecting, presence or absence of the biomarker of the present disclosure in the urine from the subject may be detected, and in the determining, when it is determined that the biomarker of the present disclosure is present, it may be determined that the subject has developed urolithiasis. In the determining, when it is determined that the biomarker of the present disclosure is absent, it may be determined that the subject has not developed urolithiasis. In the determination of the presence or absence of the biomarker of the present disclosure in the determining, for example, when the biomarker of the present disclosure is detected in the detecting, it may be determined that the biomarker of the present disclosure is present. In the determination of the presence or absence of the biomarker of the present disclosure in the determining, for example, when the biomarker of the present disclosure is not detected in the detecting, it may be determined that the biomarker of the present disclosure is absent.

The determination method of the present disclosure may include a process of determining development of urolithiasis in the subject by comparing an amount of the biomarker of the present disclosure in the urine from the subject with a reference value. The reference value is not particularly limited, and examples thereof include the amount of the biomarker of the present disclosure or the like in a healthy subject, a patient with urolithiasis, a patient with no recurrence of urolithiasis, a patient with recurrence of urolithiasis, and the like. In the case of prognosis evaluation, the reference value may be, for example, the amount of the biomarker of the present disclosure in the same subject after treatment (for example, immediately after treatment). The healthy subject is, for example, one who has never developed urolithiasis before. The patient with urolithiasis is, for example, a patient in a state of having developed urolithiasis as the initial onset. The patient with no recurrence of urolithiasis is, for example, a patient whose urolithiasis has not recurred within 5 years, or a patient whose urolithiasis has not recurred within 10 years after treatment of urolithiasis. It is known that nearly half of urolithiasis recurs in 5 years and more than half thereof recurs in 10 years. Therefore, the patient with no recurrence of urolithiasis can be referred to as, for example, a patient whose urolithiasis has remitted, or a patient whose urolithiasis has been completely cured. The patient with recurrence of urolithiasis is, for example, a patient in a state of having recurrence of urolithiasis after treatment of urolithiasis. The development and recurrence of urolithiasis can be diagnosed by, for example, an imaging examination. For the imaging examination, for example, the above description can be applied.

The reference value can be obtained, for example, using urine collected from a healthy subject, a patient with no recurrence of urolithiasis, a patient with urolithiasis, and/or a patient with recurrence of urolithiasis as described above (hereinafter also referred to as reference urine). In the case of prognosis evaluation, for example, in addition to or instead of the reference urine, reference urine collected from the same subject after treatment may be used. The reference value may be measured, for example, simultaneously with or in advance of measurement of the test urine from the subject. The latter case is preferable because, for example, it becomes unnecessary to obtain a reference value every time the test urine from the subject is measured. It is preferable that the test urine from the subject and the reference urine are, for example, collected under the same conditions, and detection of the biomarker of the present disclosure is performed under the same conditions.

In the determining, a method for evaluating development of urolithiasis in the subject is not particularly limited, and can be appropriately determined depending on the type of the reference value. As a specific example, when the amount of the biomarker of the present disclosure in the test urine from the subject is significantly higher than the amount of the biomarker of the present disclosure in the reference urine from the healthy subject or the patient with no recurrence of urolithiasis, when the amount of the biomarker of the present disclosure in the test urine from the subject is the same as the amount of the biomarker of the present disclosure in the reference urine from the patient with urolithiasis or the patient with recurrence of urolithiasis (when there is no significant difference), and/or when the amount of the biomarker of the present disclosure in the test urine from the subject is significantly higher than the amount of the biomarker of the present disclosure in the reference urine from the patient with urolithiasis or the patient with recurrence of urolithiasis, it can be evaluated that the subject has a risk or a high risk of developing urolithiasis. Further, when the amount of the biomarker of the present disclosure in the test urine from the subject is the same as the amount of the biomarker of the present disclosure in the reference urine from the healthy subject or the patient with no recurrence of urolithiasis (when there is no significant difference), when the amount of the biomarker of the present disclosure in the test urine from the subject is significantly lower than the amount of the biomarker of the present disclosure in the reference urine from the healthy subject or the patient with no recurrence of urolithiasis, and/or when the amount of the biomarker of the present disclosure in the test urine from the subject is significantly lower than the amount of the biomarker of the present disclosure in the reference urine from the patient with urolithiasis or the patient with recurrence of urolithiasis, it can be evaluated that the subject has no risk or a low risk of developing urolithiasis.

In the determining in the case of evaluating the prognosis state, for example, evaluation and judgment may be performed in the same manner as described above, or evaluation can also be performed using the amount of the biomarker of the present disclosure in the reference urine from the same subject after treatment as a reference value. As a specific example, when the amount of the biomarker of the present disclosure in the test urine from the subject is significantly higher than the reference value, it can be evaluated that the subject has a risk of recurrence or worsening after the treatment. Further, when the amount of the biomarker of the present disclosure in the test urine from the subject is the same as the reference value (when there is no significant difference) and/or is significantly lower than the reference value, it can be evaluated that the subject has no risk or a low risk of recurrence after the treatment.

In the present disclosure, for example, urine from the same subject may be collected over time, and the amounts of the biomarker of the present disclosure in the urine may be compared. This allows, for example, determining that the possibility of development has increased if the amount increases over time, and the possibility of development has decreased, or that healing has progressed if the amount decreases over time, or the like.

The determination method of the present disclosure may further include, for example, a process of administering a therapeutic agent for urolithiasis to a subject evaluated in the determining as having a risk or a high risk of developing urolithiasis, or as having a risk of recurrence or worsening after treatment (administering). In this case, the determination method of the present disclosure can also be referred to as a method for determining and treating urolithiasis.

The therapeutic agent for urolithiasis is not particularly limited, and examples thereof include a suppressing substance of the present disclosure described later (a pharmaceutical containing a formation-inhibiting substance described later), a Quercus salicina extract, and a combination of these. Administration conditions (an administration subject, a dose, a dosage form, an administration method, and the like) of the therapeutic agent for urolithiasis are not particularly limited, and can be appropriately determined depending on the type of the therapeutic agent.

The determination method of the present disclosure allows, for example, development of urolithiasis to be accurately determined. The determination method of the present disclosure further allows, for example, recurrence of urolithiasis to be accurately determined. Therefore, the determination method of the present disclosure allows, for example, determination of the possibility of development of urolithiasis, presence or absence of development of urolithiasis, prognosis evaluation for development of urolithiasis, determination of the possibility of recurrence of urolithiasis, presence or absence of recurrence of urolithiasis, prognosis evaluation for recurrence of urolithiasis, and the like.

### Suppressing substance for urolithiasis and method for suppressing urolithiasis

A suppressing substance for urolithiasis of the present disclosure (hereinafter also referred to as the suppressing substance of the present disclosure) includes a formation-inhibiting substance that inhibits formation of at least one of calcium phosphate crystals or amorphous calcium phosphate. Further, a method for suppressing urolithiasis according to the present disclosure (hereinafter also referred to as the suppressing method of the present disclosure) includes a process of administering the suppressing substance of the present disclosure to a subject. The suppressing substance and the suppressing method of the present disclosure are characterized by inhibiting formation of at least one of calcium phosphate crystals or amorphous calcium phosphate, and other configurations and conditions are not particularly limited. The suppressing substance and the suppressing method of the present disclosure can suppress development of urolithiasis. For the suppressing substance and the suppressing method of the present disclosure, for example, the descriptions of the biomarker of the present disclosure and the determination method of the present disclosure can be applied.

Examples of the formation-inhibiting substance include a substance that adjusts urine pH, and a substance that chelates an inorganic component. The substance that chelates an inorganic component may be, for example, sodium citrate or the like. The formation-inhibiting substance may be, for example, a diuretic, a uric acid production inhibitor, or the like. The diuretic may be, for example, a thiazide diuretic or the like. The uric acid production inhibitor may be, for example, allopurinol, febuxostat, or the like. The formation-inhibiting substance can be used, for example, as a contained component in a food, a beverage, a food additive, a beverage additive, a supplement, a pharmaceutical, and the like. Further, the formation-inhibiting substance can be used, for example, as a food, a beverage, a food additive, a beverage additive, a supplement, a pharmaceutical, or the like itself.

Examples of the suppressing substance include a food containing the formation-inhibiting substance, a beverage containing the formation-inhibiting substance, a food additive containing the formation-inhibiting substance, a beverage additive containing the formation-inhibiting substance, a supplement containing the formation-inhibiting substance, and a pharmaceutical containing the formation-inhibiting substance.

When the suppressing substance of the present disclosure is a food containing the formation-inhibiting substance, the food is not particularly limited, and examples thereof include processed grain foods, processed vegetable foods, processed fruit foods, processed meat products, processed seafood products, dairy products, and confectionery. Examples of the confectionery include candies, drops, gummies, Ramune soft candy, snack confections, baked confections, and steamed confections.

When the suppressing substance of the present disclosure is a pharmaceutical containing the formation-inhibiting substance, an administration form of the pharmaceutical is not particularly limited. Further, a dosage form of the pharmaceutical is not particularly limited, and can be appropriately determined depending on, for example, the administration form. Further, in the pharmaceutical, a compounding amount of the formation-inhibiting substance is not particularly limited. Further, in the pharmaceutical, administration conditions of the formation-inhibiting substance are not particularly limited.

### Method for screening candidate substance for suppressing urolithiasis

A method for screening a candidate substance for suppressing urolithiasis according to the present disclosure (hereinafter also referred to as the screening method of the present disclosure) is not particularly limited, and is, for example, a screening method including a process of selecting, from test substances, a formation-inhibiting substance that inhibits formation of at least one of calcium phosphate crystals or amorphous calcium phosphate as a candidate substance for suppressing urolithiasis. The screening method of the present disclosure is characterized by selecting the candidate substance for suppressing urolithiasis based on the biomarker of the present disclosure in the selecting, and other processes and conditions are not particularly limited. For the screening method of the present disclosure, for example, the descriptions of the biomarker of the present disclosure, the determination method of the present disclosure, and the suppressing substance and the suppressing method of the present disclosure can be applied.

Examples of the formation-inhibiting substance include a substance that adjusts urine pH, and a substance that chelates an inorganic component. The substance that chelates an inorganic component may be, for example, sodium citrate or the like. The formation-inhibiting substance may be, for example, a formation-inhibiting substance for foods, a formation-inhibiting substance for beverages, a formation-inhibiting substance for food additives, a formation-inhibiting substance for beverages, a formation-inhibiting substance for supplements, a formation-inhibiting substance for pharmaceuticals, or the like.

The screening method of the present disclosure may be, for example, a screening method including performing insolubilization processing in the presence of the test substances to form at least one of the calcium phosphate crystals or the amorphous calcium phosphate, and detecting at least one of the calcium phosphate crystals or the amorphous calcium phosphate, wherein in the selecting, the candidate substance for suppressing urolithiasis is selected from the test substances based on a result of the detection of at least one of the calcium phosphate crystals or the amorphous calcium phosphate. The insolubilization processing is not particularly limited, and for example, the above description can be applied. Further, a method for detecting the biomarker according to the present disclosure is not particularly limited, and for example, the above description can be applied.

The screening method of the present disclosure may be, for example, a screening method including the detecting in which presence or absence of at least one of the calcium phosphate crystals or the amorphous calcium phosphate is detected, and the selecting in which the test substance, for which at least one of the calcium phosphate crystals or the amorphous calcium phosphate is determined to be absent, is selected as the candidate substance for suppressing urolithiasis. The insolubilization processing is not particularly limited, and for example, the above description can be applied. Further, a method for detecting the biomarker according to the present disclosure is not particularly limited, and for example, the above description can be applied.

The screening method of the present disclosure may be, for example, a screening method including the detecting in which an amount of at least one of the calcium phosphate crystals or the amorphous calcium phosphate is detected, and the selecting in which the test substance, for which the amount of at least one of the calcium phosphate crystals or the amorphous calcium phosphate is lower than that of a control in the absence of the test substance, is selected as the candidate substance for suppressing urolithiasis. The insolubilization processing is not particularly limited, and for example, the above description can be applied. Further, a method for detecting the biomarker according to the present disclosure is not particularly limited, and for example, the above description can be applied.

In the screening method of the present disclosure, for example, in the performing insolubilization processing, the insolubilization processing may be supersaturation. For the insolubilization processing by the supersaturation, for example, the above description can be applied.

In the screening method of the present disclosure, for example, in the performing insolubilization processing, the supersaturation may be cooling. For the supersaturation by the cooling, for example, the above description can be applied.

The test substance is not particularly limited, and may be, for example, at least one selected from the group consisting of low-molecular-weight compounds, peptides, and proteins.

### Use of formation-inhibiting substance

The present disclosure provides a formation-inhibiting substance that inhibits formation of at least one of calcium phosphate crystals or amorphous calcium phosphate, or use thereof, for use in treating urolithiasis. The present disclosure provides a formation-inhibiting substance that inhibits formation of at least one of calcium phosphate crystals or amorphous calcium phosphate, or use thereof, for use in producing a therapeutic agent for urolithiasis.

Use of at least one of calcium phosphate crystals or amorphous calcium phosphate in urine

The present disclosure provides use of at least one of calcium phosphate crystals or amorphous calcium phosphate in urine for determining development of urolithiasis. The present disclosure provides use of at least one of calcium phosphate crystals or amorphous calcium phosphate in urine for determining recurrence of urolithiasis. The present disclosure provides use of at least one of calcium phosphate crystals or amorphous calcium phosphate in urine for determining early recurrence of urolithiasis.

### Examples

Next, Examples of the present disclosure will be described below. It is to be noted, however, that the present disclosure is not limited to the following Examples. Commercial reagents were used based on their protocols unless otherwise specified.

### [Example 1]

It was verified that at least one of calcium phosphate crystals or amorphous calcium phosphate can be formed by cooling a urine sample.

### (1) Effect of temperature conditions on formation of insolubilized material

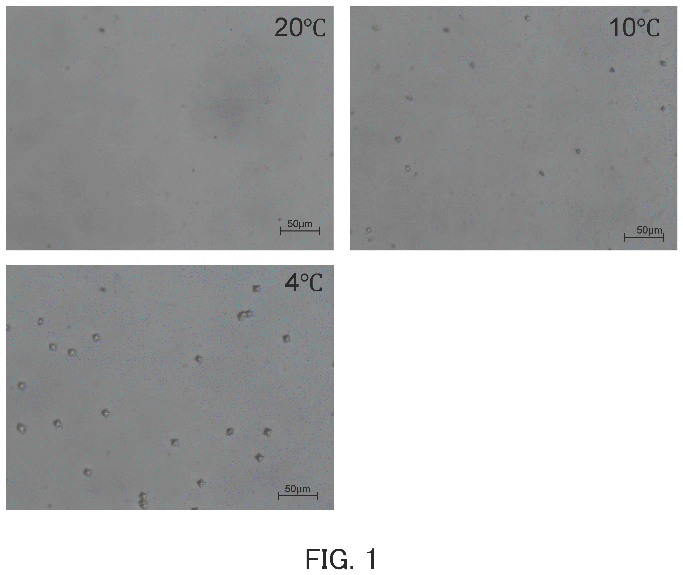

A urine sample was collected from a patient with urolithiasis in whom calcium oxalate calculi were detected. The collected urine sample was cooled under temperature conditions of 20°C, 10°C, and 4°C for 24 hours or more in a state of being left standing. The cooled urine sample was loaded on a slide glass, and a cover glass was placed directly above the loaded urine sample to prepare a specimen. The specimen was microscopically examined using an optical microscope under conditions of no staining and a 200-fold magnified field of view. As a result, as shown in FIG. 1, the lower the cooling temperature was, the larger the size and the larger the number of calcium oxalate crystals were. Therefore, subsequent Examples were performed by setting the cooling temperature to 4°C.

### (2) Basic data of patients with urolithiasis

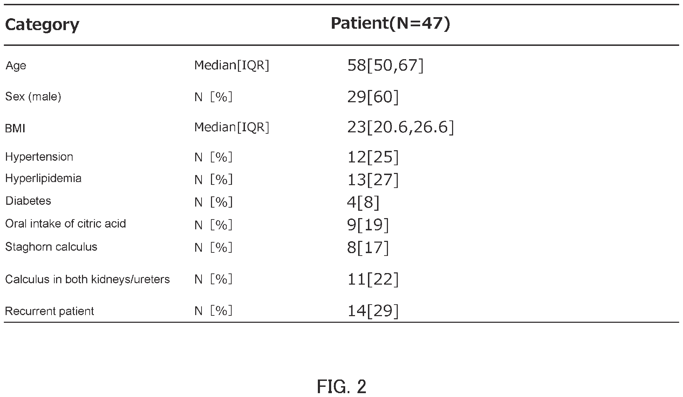

47 patients with urolithiasis shown in FIG. 2 were recruited as subjects in Example 1. Among the subjects, 14 patients with recurrence of urolithiasis were patients with recurrence of urolithiasis among patients for whom the absence of residual urinary calculi had been verified by an imaging examination (ultrasonography examination, plain X-ray examination, or plain CT examination) after spontaneous calculus passage, after calculus passage by treatment, or after lithotripsy by treatment. Further, the subjects were patients with urolithiasis excluding in advance patients with urolithiasis whose urinary calculi were determined to be cystine calculi, uric acid calculi, or magnesium ammonium phosphate calculi by calculus analysis. That is, the subjects were patients with urolithiasis whose urinary calculi were determined to be calcium oxalate calculi, calcium phosphate calculi, or mixed calculi of calcium oxalate calculi and calcium phosphate calculi by calculus analysis.

### (3) Observation using optical microscope

A urine sample collected in a 24-hour urine collection test of each of the subjects was centrifuged under conditions of 2500 rpm for 10 minutes, and the supernatant after centrifugation was collected. The supernatant was filtered through a filter having a pore size of 0.45 µm to remove impurities from the supernatant, thereby obtaining a filtrate. The filtrate was cooled under conditions of 4°C for 24 hours or more. The cooled filtrate was loaded on a slide glass, and a cover glass was placed directly above the loaded filtrate to prepare a specimen. The specimen was microscopically examined using an optical microscope under conditions of no staining and a high-magnification field of view (400-fold) to verify the presence or absence of an insolubilized material in the cooled filtrate. Further, types of the insolubilized materials in the cooled filtrate were morphologically classified based on the shape, color tone, size, and the like thereof.

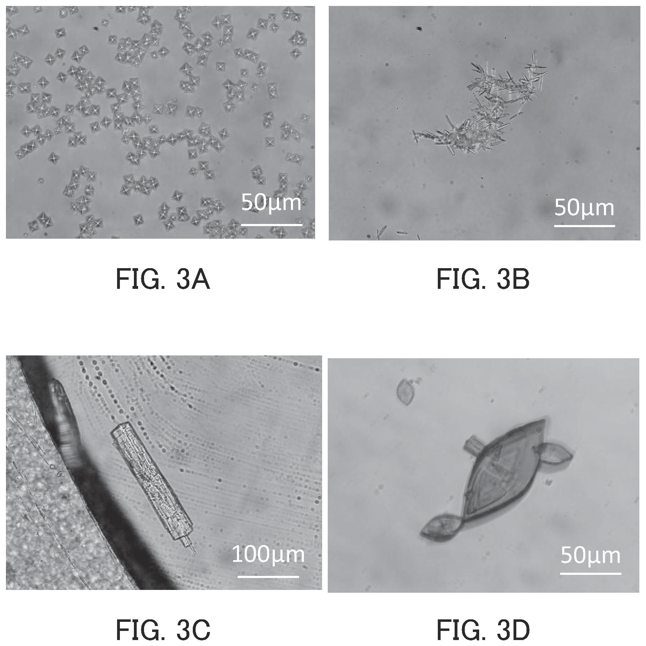

As shown in FIG. 3, it was verified that various insolubilized materials appeared in the filtrate by cooling the filtrate at 4°C for 24 hours or more. As a result of morphologically classifying the types of the insolubilized materials, the insolubilized material A was classified as a calcium oxalate crystal because it was a colorless crystal having a regular octahedral shape. Further, the insolubilized materials B and C were classified as calcium phosphate crystals because they were colorless to grayish white crystals having rosette-like or plate-like shapes. Further, the insolubilized material D was classified as a uric acid crystal because it was a yellowish-brown crystal having a rhombic shape.

### (4) Raman spectroscopic analysis

The cooled filtrate was loaded on a slide glass with a silicon sheet, and a quartz cover glass was placed directly above the loaded filtrate to prepare a specimen. The specimen was microscopically examined using a micro-Raman spectrometer. Laser irradiation was performed on the insolubilized material in the cooled filtrate, thereby classifying types of the insolubilized materials in the cooled filtrate based on a peak in a Raman shift obtained by spectroscopy of Raman scattered light. The laser irradiation was performed under the following conditions: excitation wavelength: 532 nm; exposure time: 10 seconds; number of accumulations: 6 times; laser output: 5 to 10 mW.

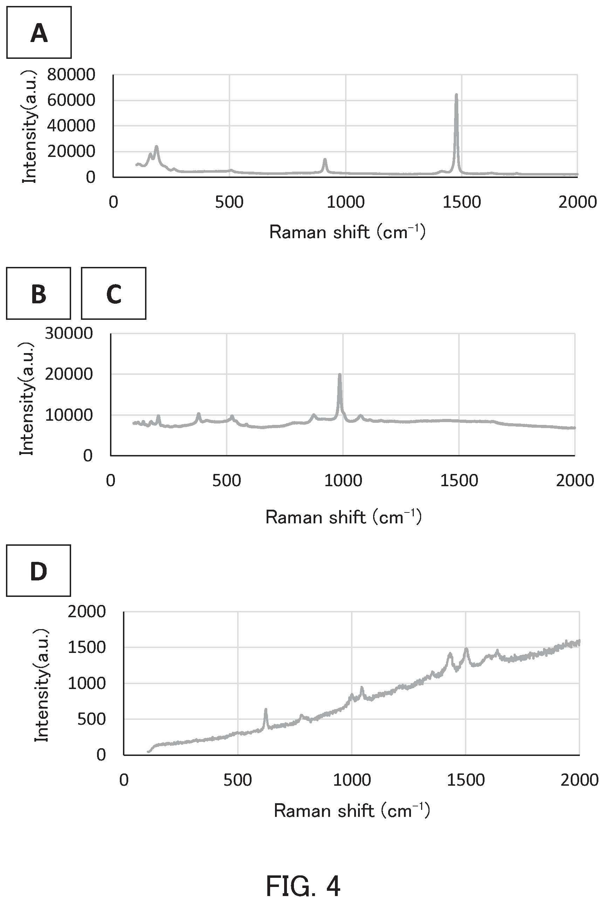

As shown in FIG. 4, as a result of analyzing the Raman scattered light of the insolubilized material by Raman spectroscopy, the insolubilized material A was found to contain calcium oxalate dihydrate because of the peaks in Raman shifts around 910 cm⁻¹ and around 1479 cm⁻¹ being detected. The insolubilized materials B and C were found to contain calcium hydrogen phosphate (calcium monohydrogen phosphate dihydrate (Brushite)), because of the peaks in Raman shifts around 990 cm⁻¹ and around 878 cm⁻¹ being detected. The insolubilized material D was found to contain uric acid, because of the peaks in Raman shifts around 623 cm⁻¹, around 1004 cm⁻¹, around 1431 cm⁻¹, and around 1500 cm⁻¹ being detected.

From the results of Examples 1 (3) and (4), the 47 patients with urolithiasis were classified into 26 patients (55%) in whom calcium oxalate crystals were detected, 6 patients (13%) in whom calcium phosphate crystals were detected, 2 patients (4%) in whom uric acid crystals were detected, and 13 patients (28%) in whom no crystals were detected.

From the above results, it was found that at least one of calcium phosphate crystals or amorphous calcium phosphate can be formed by the insolubilization processing of cooling a urine sample. Further, it was found that at least one of calcium oxalate crystals or amorphous calcium oxalate can be formed by the insolubilization processing of cooling a urine sample. Further, it was found that at least one of uric acid crystals or amorphous uric acid can be formed by the insolubilization processing of cooling a urine sample. Therefore, it was presumed that at least one of inorganic component crystals or an amorphous inorganic component can be formed by the insolubilization processing of cooling a urine sample.

### [Example 2]

The relationship between the presence or absence of an insolubilized material and recurrence of urolithiasis was examined, and it was verified that, when at least one of calcium phosphate crystals or amorphous calcium phosphate was detected, a possibility of recurrence of urolithiasis was high.

45 patients with urolithiasis, excluding 2 subjects in whom an insolubilized material of uric acid was detected from the subjects in Example 1, were recruited as subjects in Example 2. The subjects in Example 2 were classified into three groups consisting of 13 subjects in whom no insolubilized material was detected, 26 subjects in whom an insolubilized material of calcium oxalate was detected, and 6 subjects in whom an insolubilized material of calcium phosphate was detected. For the three groups, the relationship between the presence or absence of an insolubilized material and recurrence of urolithiasis was examined.

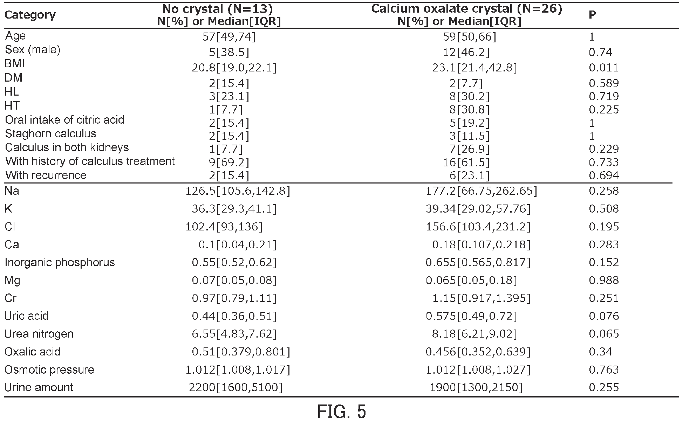

As shown in FIG. 5, there was no statistically significant difference in the number of patients with recurrence of urolithiasis (with recurrence) between the group in whom no insolubilized material was detected (no crystals) and the group in whom an insolubilized material of calcium oxalate was detected (calcium oxalate crystals). Further, the value of BMI was statistically significantly higher in the group in whom an insolubilized material of calcium oxalate was detected, than in the group in whom no insolubilized material was detected. In contrast, there was no statistically significant difference in other evaluation items between the group in whom no insolubilized material was detected and the group in whom an insolubilized material of calcium oxalate was detected.

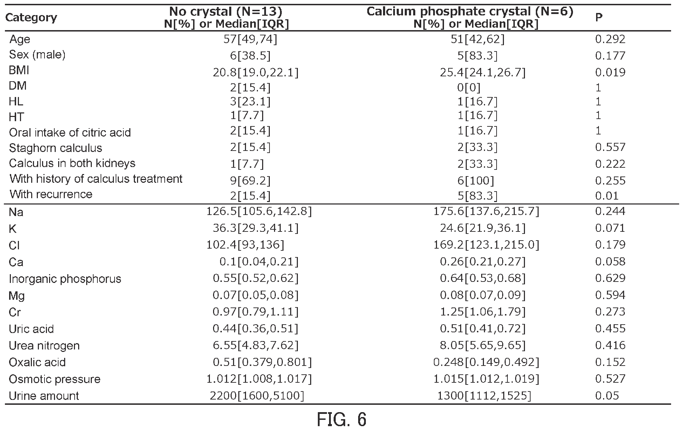

As shown in FIG. 6, the number of patients with recurrence of urolithiasis (with recurrence) was statistically significantly larger in the group in whom an insolubilized material of calcium phosphate was detected (calcium phosphate crystals), than in the group in whom no insolubilized material was detected (no crystals). Further, the value of BMI was statistically significantly higher in the group in whom an insolubilized material of calcium phosphate was detected, than in the group in whom no insolubilized material was detected. In contrast, there was no statistically significant difference in other evaluation items between the group in whom no insolubilized material was detected and the group in whom an insolubilized material of calcium phosphate was detected.

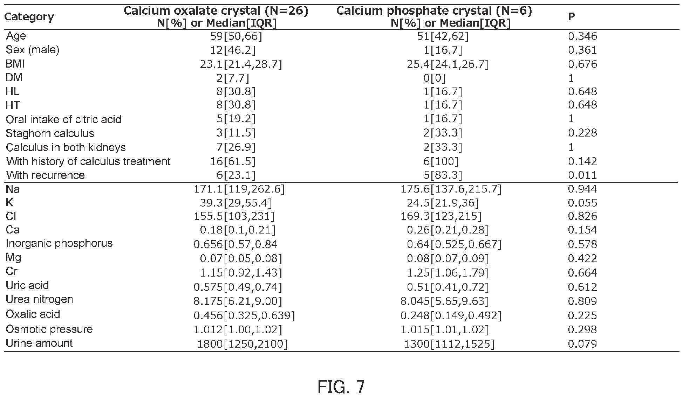

As shown in FIG. 7, the number of patients with recurrence of urolithiasis (with recurrence) was statistically significantly larger in the group in whom an insolubilized material of calcium phosphate was detected (calcium phosphate crystals), than in the group in whom an insolubilized material of calcium oxalate was detected (calcium oxalate crystals). In contrast, there was no statistically significant difference in other evaluation items between the group in whom an insolubilized material of calcium oxalate was detected and the group in whom an insolubilized material of calcium phosphate was detected.

From the above results, it was found that, when at least one of calcium phosphate crystals or amorphous calcium phosphate was detected, a possibility of recurrence of urolithiasis was high. In contrast, it was found that, when at least one of calcium oxalate crystals or amorphous calcium oxalate was detected, the presence or absence of recurrence of urolithiasis could not be differentiated.

### [Example 3]

The structure and components of calcium oxalate calculi collected from patients with urolithiasis were analyzed, and it was verified that one of calcium phosphate crystals or amorphous calcium phosphate plays a role as a starting point of calculus growth.

A calcium oxalate calculus was collected from the kidney of a 60-year-old male patient with urolithiasis. The calcium oxalate calculus was mainly composed of calcium oxalate dihydrate (COD) crystals. The calcium oxalate calculus was immersed in a calcium oxalate solution to induce a solution-mediated phase transition. By the solution-mediated phase transition, the composition of the inner layer and the outermost layer of the calcium oxalate calculus underwent a phase transition to calcium oxalate monohydrate (COM) crystals. A pretreatment was performed on the calcium oxalate calculus in which the solution-mediated phase transition was induced, and a thin slice having a thickness of 20 to 30 µm was prepared from the pretreated calcium oxalate calculus using a resin embedding method.

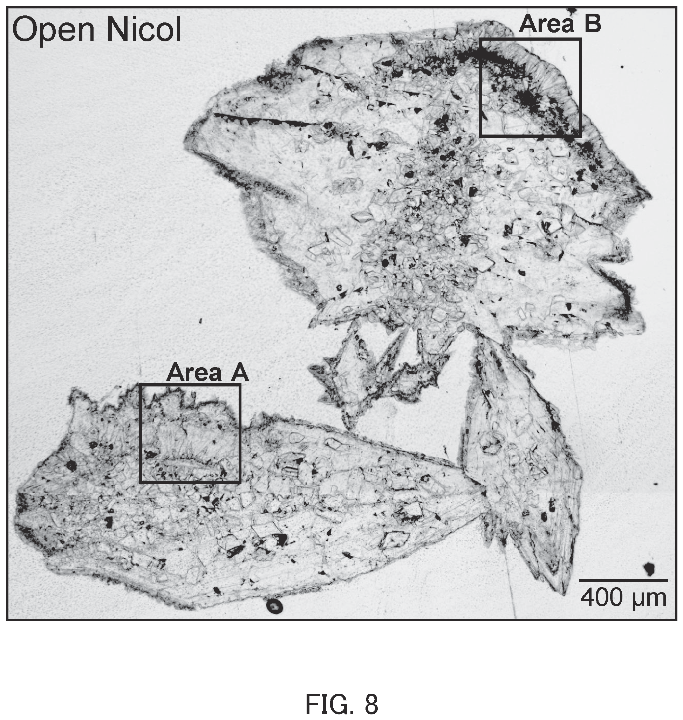

FIG. 8 is an observation image of the thin slice by a polarizing microscope (OPTIPHOT2-POL, manufactured by NIKON CORPORATION) under an open-nicol condition. An examination was performed on Area A and Area B in FIG. 8 in which co-localization of a phosphorus element and a zinc element was detected in elemental mapping on the thin slice by XRF analysis to be described later.

### Area A

### (1) XRF analyzer

The thin slice was analyzed by X-ray fluorescence (XRF). Specifically, positions of four elements (Ca, P, Zn, Si) on the thin slice were identified using an XRF analyzer (XGT9000, manufactured by HORIBA, Ltd.). In all measurements, the voltage of the X-ray tube was set to 30 kV, and the current of the X-ray tube was set to 1000 µA.

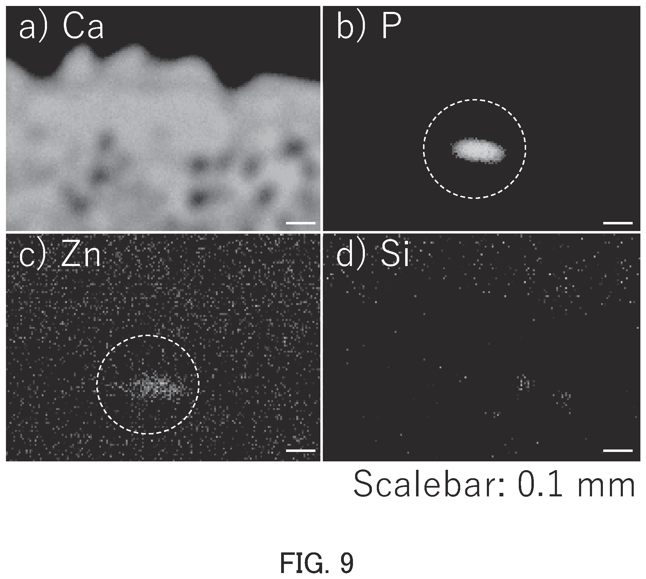

As shown in FIG. 9, as a result of analyzing the four elements on the thin slice in Area A using the XRF analyzer, it was found that calcium elements were distributed throughout the calculus. In contrast, it was found that phosphorus elements were concentrated and localized in a specific region, as shown by a circle drawn with a dashed line. Further, it was found that zinc elements were also concentrated and localized in the localized region of the phosphorus elements, as shown by the circle drawn with the dashed line. Therefore, it was found that the phosphorus element and the zinc element were co-localized in a specific region in Area A.

### (2) Observation using polarizing microscope under cross-nicol condition

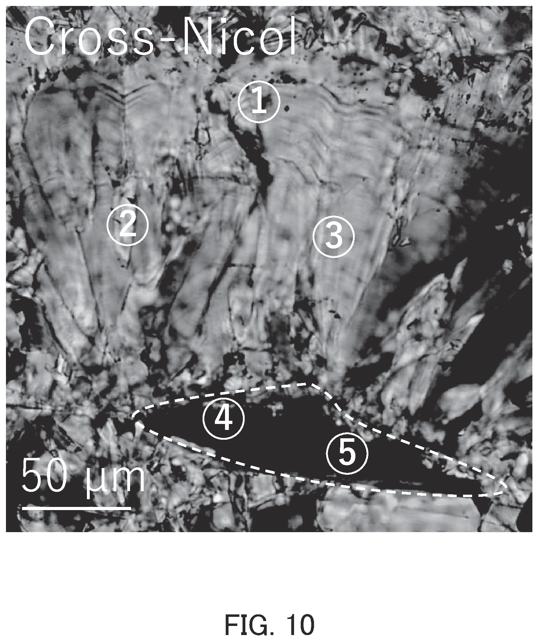

As a result of observing Area A in FIG. 8 using a polarizing microscope under a cross-nicol condition, as shown in FIG. 10, regions with layered structures (indicated by circle 2 and circle 3) and dark regions that do not transmit light (indicated by circle 4 and circle 5, and a boundary line drawn with a dashed line) were observed. In observation using a polarizing microscope under a cross-nicol condition, it is known that an aggregate of microcrystals scatters light at its boundary surface, and thus is observed as a dark region. Therefore, the dark region was considered to be a structure in which some kind of microcrystals aggregated. Further, when an elemental distribution in the dark region was examined by the elemental mapping result in the (1) XRF analyzer, it was found that the dark region coincided with the region where the phosphorus element and the zinc element were concentrated and co-localized.

### (3) Laser Raman microscope

In order to identify the types of each layer on the thin slice in more detail, the thin slice was observed using a laser Raman microscope (RAMAN touch, manufactured by Nanophoton). Specifically, by detecting specific peaks related to chemical bonds peculiar to each layer using the laser Raman microscope, a COD phase, a COM phase, and a calcium phosphate phase in the thin slice were identified. The measurement conditions of the laser Raman microscope were as follows: laser wavelength: 787 nm; laser output: 1.50 ± 0.25 mW; number of grooves of a diffraction grating: 600 gr/mm; measurement wavenumber range: 600 to 1600 cm⁻¹.

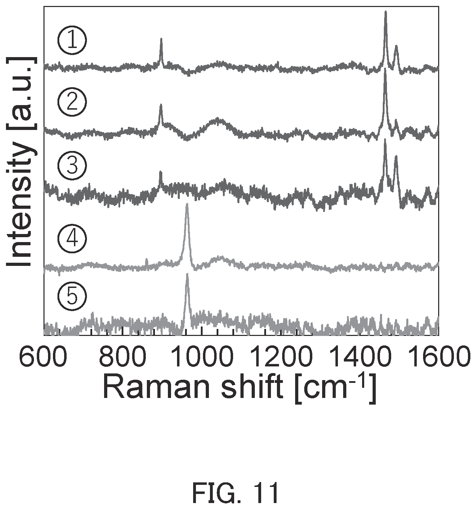

Points indicated by circle 1 to circle 5 on the thin slice shown in FIG. 10 were analyzed using the laser Raman microscope. As a result, as shown in FIG. 11, at the points indicated by circle 1 to circle 3 (the regions with layered structures in Area A), a peak peculiar to the COM phase was detected. In contrast, at the points indicated by circle 4 and circle 5 which are the dark regions, a peak peculiar to a calcium phosphate phase, particularly hydroxyapatite, was detected. Therefore, it was found that the dark regions contained an aggregate of calcium phosphate microcrystals.

### (4) Immunofluorescence staining

In order to visualize a distribution of protein in the thin slice, immunofluorescence staining was performed on the thin slice, and the stained thin slice was observed using a fluorescence microscope (A1R, manufactured by NIKON CORPORATION). The immunostaining targeted a protein called osteopontin (OPN). Note that OPN is known as a calcium-binding protein, and it has been reported to inhibit or promote formation of urinary calculi.

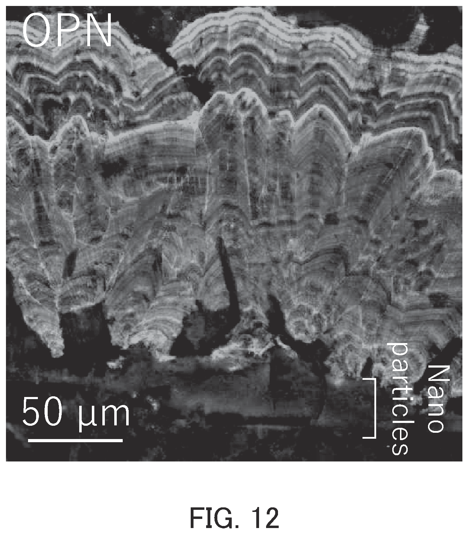

As shown in FIG. 12, as a result of immunofluorescence staining of the thin slice, the striped structure of OPN was visualized more clearly in the outer layer than in the inner layer. Further, since the structure of the aggregate of calcium phosphate microcrystals contained in the dark region could not be verified on a micrometer-order scale, its size was considered to be on a nanometer order.

From the above results, it was found that the dark region on the thin slice contained an aggregate of calcium phosphate microcrystals in which a zinc element was co-localized. In urolithiasis, it is known that a crystal nucleus called Randall's plaque is formed in the interstitium of the renal papilla, and the Randall's plaque serves as a scaffold for calculus formation, and crystals grow. In past studies, it has been reported that Randall's plaque is an aggregate of calcium phosphate microcrystals on a nanometer order, and involves zinc co-localization. Therefore, it was presumed that the dark region was Randall's plaque.

Further, in FIG. 10, from the dark region, the COM phase, which is the region with a layered structure in Area A, extends outward. In past studies, Randall's plaque is known to serve as a scaffold for calculus formation. Therefore, the result was presumed to further support that Randall's plaque plays a role as a starting point of nucleation of COM crystals.

### Area B

### (1) XRF analysis

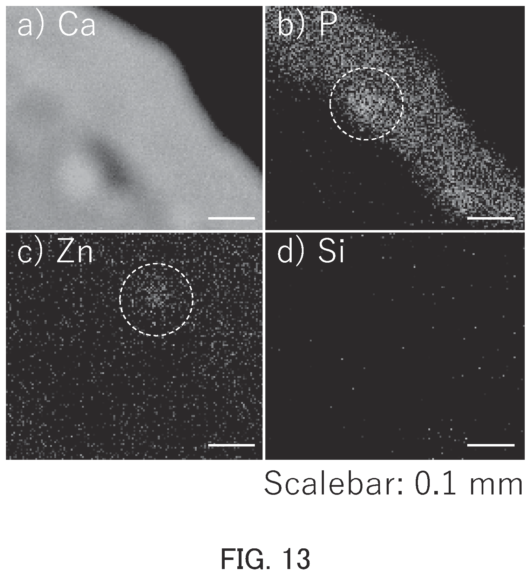

Area B on the thin slice was analyzed by XRF by the same method as Area A. As shown in FIG. 13, as a result of analyzing the four elements on the thin slice in Area B using the XRF analyzer, it was found that calcium elements were distributed throughout the calculus. In contrast, it was found that phosphorus elements were concentrated and localized in a specific region, as shown by a circle drawn with a dashed line. Further, it was found that zinc elements were also concentrated and localized in the localized region of the phosphorus elements, as shown by the circle drawn with the dashed line. Therefore, it was found that the phosphorus element and the zinc element were co-localized in a specific region in Area B.

### (2) Observation using polarizing microscope under cross-nicol condition

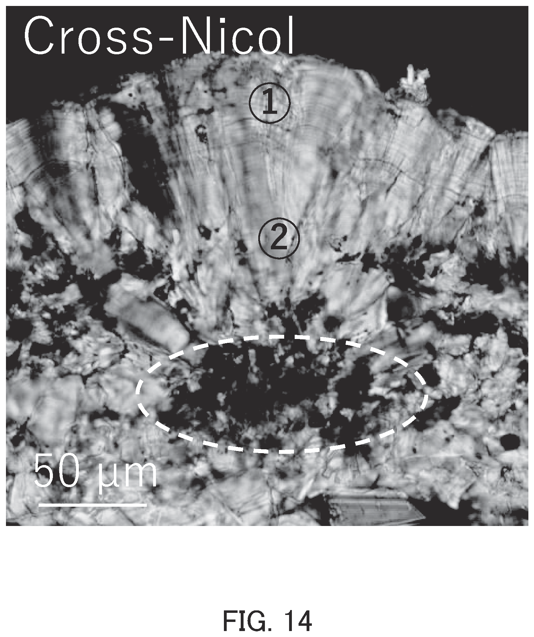

As a result of observing Area B in FIG. 8 using a polarizing microscope under a cross-nicol condition, as shown in FIG. 14, regions with layered structures (indicated by circle 1 and circle 2) and a dark region that slightly transmits light (indicated by a circle drawn with a dashed line) were observed. Further, when an elemental distribution in the dark region that slightly transmits light was examined by the elemental mapping result in the (1) XRF analyzer, it was found that the dark region that slightly transmits light coincided with the region where the phosphorus element and the zinc element were concentrated and co-localized.

### (3) Laser Raman microscope

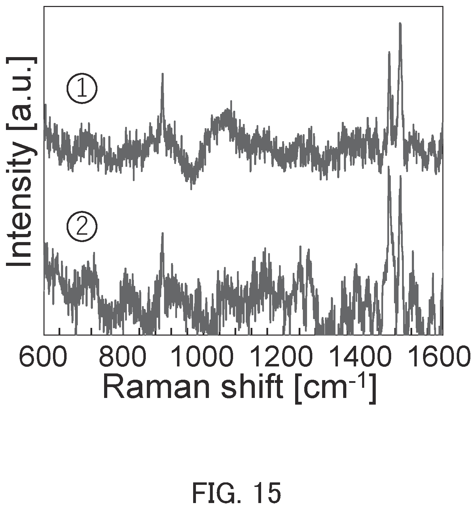

Area B on the thin slice was observed using a laser Raman microscope by the same method as Area A. Points indicated by circle 1 and circle 2 (the regions with layered structures) and the dark region that slightly transmits light on the thin slice shown in FIG. 14 were analyzed using the laser Raman microscope. As a result, as shown in FIG. 15, at the points indicated by circle 1 and circle 2 (the regions with layered structures), a peak peculiar to the COM phase was detected. However, in the dark region that slightly transmits light, a Raman spectrum could not be obtained due to the influence of background fluorescence. Therefore, it was presumed that the dark region that slightly transmits light contained some kind of protein emitting autofluorescence.

### (4) Immunofluorescence staining

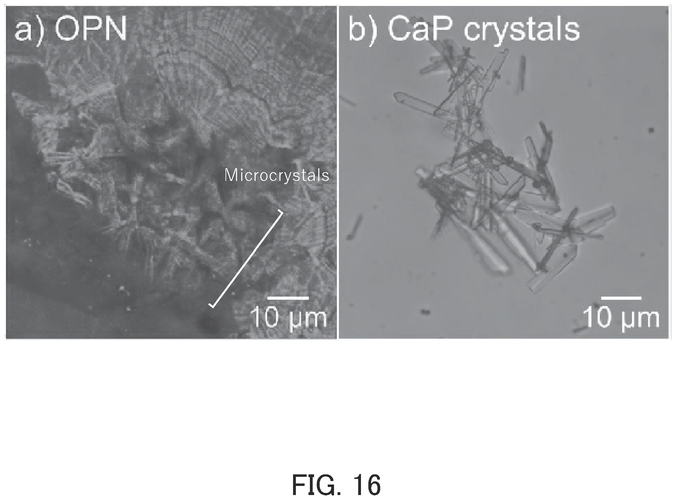

In Area B on the thin slice, immunofluorescence staining was performed by the same method as Area A, and it was verified whether the dark region that slightly transmits light contained OPN, which is a protein. As shown in a) of FIG. 16, as a result of immunofluorescence staining of the thin slice, an aggregate of plate-like crystals containing OPN was observed in the dark region that slightly transmits light. Further, the region where the aggregate of plate-like crystals containing OPN was observed coincided with the region where the phosphorus element and the zinc element were co-localized in the XRF analysis. Further, as shown in a) of FIG. 16, the aggregate of plate-like crystals containing OPN was very similar in morphology to the calcium phosphate crystals shown in b) of FIG. 16 (observed in a urine sample with an optical microscope), and its size was on a micrometer order. Therefore, it was found that the dark region that slightly transmits light was a calcium phosphate phase in which a zinc element was co-localized, but was not Randall's plaque from its size.

From the above results, it was found that the dark region that slightly transmits light on the thin slice contained the aggregate of calcium phosphate crystals on a micrometer order, which was not Randall's plaque. Further, in FIG. 13, from the dark region that slightly transmits light, the COM phase, which is the region with a layered structure, extends outward. Therefore, it was presumed that one of calcium phosphate crystals or amorphous calcium phosphate plays a role as a starting point of nucleation of COM crystals, even if it is not Randall's plaque.

Further, the aggregate of calcium phosphate crystals on a micrometer order contained OPN, which is a protein. Therefore, it was presumed that organic components such as proteins contained in urine have an influence on the formation of one of calcium phosphate crystals or amorphous calcium phosphate.

### [Example 4]

The relationship between the presence or absence of recurrence of urolithiasis and an insolubilized material was examined, and it was verified that a possibility of detection of at least one of calcium phosphate crystals or amorphous calcium phosphate was high in a patient with recurrence of urolithiasis.

### (1) Examination of relationship between presence or absence of recurrence of urolithiasis and insolubilized material

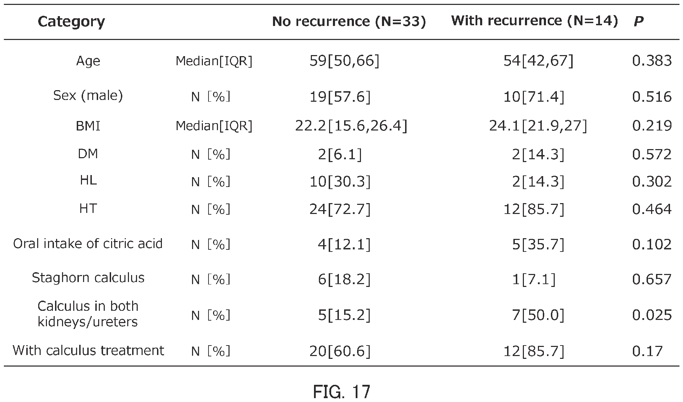

47 patients with urolithiasis shown in FIG. 17 (the same patients with urolithiasis as in Example 1) were recruited as subjects in Example 4. The subjects in Example 4 were classified into 2 groups consisting of 33 patients with no recurrence of urolithiasis (without recurrence) and 14 patients with recurrence of urolithiasis (with recurrence). In the patients with recurrence of urolithiasis, the number of cases in which urinary calculi were present in both kidneys or both ureters was statistically significantly greater than that in the patients with no recurrence of urolithiasis. For the 2 groups, the relationship between the presence or absence of recurrence of urolithiasis and an insolubilized material was examined.

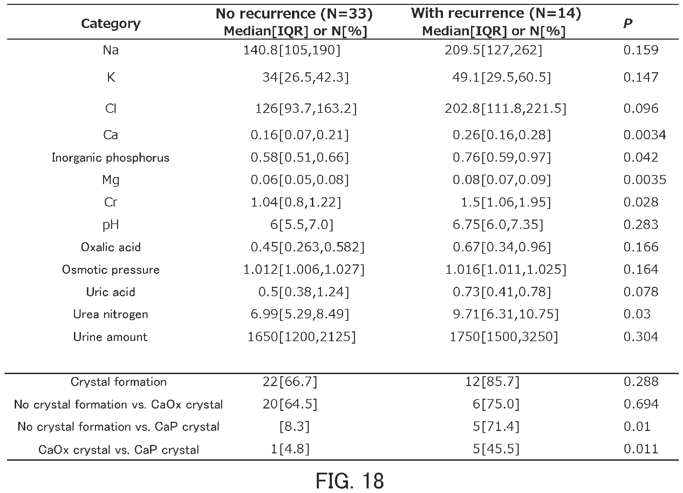

As shown in "No crystal formation vs. CaP crystal" in FIG. 18, compared to the subjects in whom no insolubilized material was detected (no crystal formation), the number of patients with recurrence of urolithiasis was statistically significantly larger in the subjects in whom an insolubilized material of calcium phosphate was detected (CaP crystals). Further, as shown in "CaOx crystal vs. CaP crystal" in FIG. 18, compared to the subjects in whom insolubilization of calcium oxalate was detected (CaOx crystals), the number of patients with recurrence of urolithiasis was statistically significantly larger in the subjects in whom insolubilization of calcium phosphate was detected (CaP crystals). In contrast, as shown in "With crystal formation" in FIG. 18, there was no statistically significant difference in the number of subjects in whom an insolubilized material of calcium oxalate, an insolubilized material of calcium phosphate, or an insolubilized material of uric acid was detected (with crystal formation) between the group of patients with no recurrence of urolithiasis and the group of patients with recurrence of urolithiasis. Further, as shown in "No crystal formation vs. CaOx crystal" in FIG. 18, there was no statistically significant difference in the number of patients with recurrence of urolithiasis between the subjects in whom no insolubilized material was detected (no crystal formation) and the subjects in whom an insolubilized material of calcium oxalate was detected (CaOx crystals).

Further, as shown in FIG. 18, values of evaluation items related to renal function (Ca, inorganic phosphorus, Mg, Cre, urea nitrogen) were statistically significantly higher in the group of patients with recurrence of urolithiasis than in the group of patients with no recurrence of urolithiasis.

From the above results, it was found that a possibility that at least one of calcium phosphate crystals or amorphous calcium phosphate is detected was high in a patient with recurrence of urolithiasis. In contrast, it was found that a possibility that at least one of calcium oxalate crystals or amorphous calcium oxalate is detected was not remarkably high in a patient with recurrence of urolithiasis.

### (2) Evaluation of insolubilized material of calcium phosphate as biomarker for determining recurrence of urolithiasis

In the same manner as in Example 4 (1), the 47 subjects in Example 1 were recruited as subjects in Example 4 (2). The subjects in Example 4 (2) consisted of 33 patients with no recurrence of urolithiasis and 14 patients with recurrence of urolithiasis. Data of the subjects in Example 4 (2) was analyzed by logistic regression analysis to search for a recurrence risk marker for urolithiasis. In the logistic regression analysis, the presence or absence of recurrence of urolithiasis was used as an objective variable, and the presence or absence of a urinary calculus in both kidneys or both ureters, results of a 24-hour urine collection test (Ca, Mg, Cre, urea nitrogen), and the presence or absence of an insolubilized material (presence or absence of an insolubilized material of calcium phosphate) were used as explanatory variables.

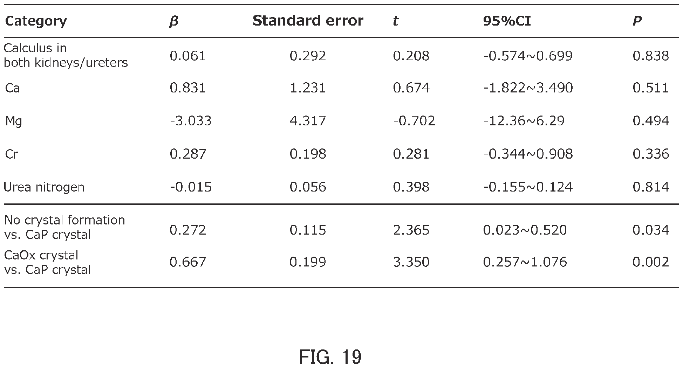

As shown in "No crystal formation vs. CaP crystal" in FIG. 19, the presence or absence of an insolubilized material of calcium phosphate was a factor that statistically significantly influenced the presence or absence of recurrence of urolithiasis when comparing the subjects in whom no insolubilized material was detected (no crystal formation) and the subjects in whom an insolubilized material of calcium phosphate was detected (CaP crystals). Further, as shown in "CaOx crystal vs. CaP crystal" in FIG. 19, the presence or absence of an insolubilized material of calcium phosphate was a factor that statistically significantly influenced the presence or absence of recurrence of urolithiasis when comparing the subjects in whom an insolubilized material of calcium oxalate was detected (CaOx crystals) and the subjects in whom an insolubilized material of calcium phosphate was detected (CaP crystals). In contrast, values or the presence or absence of other evaluation items could not be verified to have a statistically significant influence on the presence or absence of recurrence of urolithiasis.

From the above results, it was found that at least one of calcium phosphate crystals or amorphous calcium phosphate can be used as a biomarker for determining recurrence of urolithiasis. Further, it was found that at least one of calcium phosphate crystals or amorphous calcium phosphate can be used in a method for determining recurrence of urolithiasis, which determines recurrence of urolithiasis in a subject based on a detection result of at least one of calcium phosphate crystals or amorphous calcium phosphate. In patients with recurrence of urolithiasis, it is known that the same type of urinary calculus as that at the initial onset tends to occur. Therefore, based on the results of the above Examples, it was presumed that at least one of calcium phosphate crystals or amorphous calcium phosphate can be used as a biomarker for determining development of urolithiasis, or can be used in a method for determining development of urolithiasis, which determines development of urolithiasis in a subject based on a detection result of at least one of calcium phosphate crystals or amorphous calcium phosphate.

### [Example 5]

The relationship between the timing of recurrence of urolithiasis and an insolubilized material was examined, and it was verified that a possibility that at least one of calcium phosphate crystals or amorphous calcium phosphate is detected was high in a patient with early recurrence of urolithiasis. In particular, it was verified that, in a patient with early recurrence of urolithiasis, a possibility that crystals of calcium monohydrogen phosphate dihydrate (Brushite) are detected was high.

### (1) Basic data

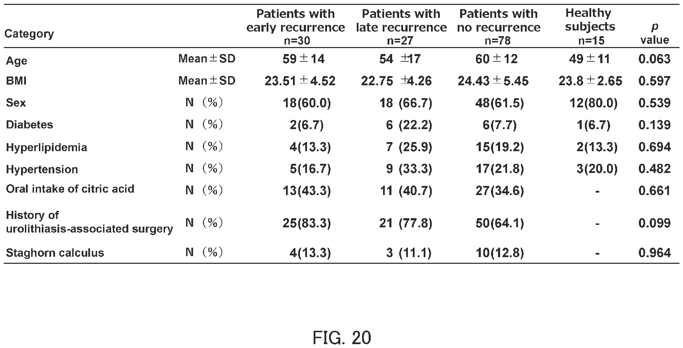

135 patients with urolithiasis and 15 healthy subjects shown in FIG. 20 were recruited as subjects in Example 5. Among the 135 patients with urolithiasis, 30 patients (22.2%) were patients with early recurrence whose urolithiasis recurred within 5 years, 27 patients (20.0%) were patients with late recurrence whose urolithiasis recurred after more than 5 years, and 78 patients (57.8%) were patients with no recurrence whose urolithiasis did not recur. As shown in FIG. 20, the subjects in Example 5 were classified into 4 groups consisting of a group of the patients with early recurrence (patients with early recurrence), a group of the patients with late recurrence (patients with late recurrence), a group of the patients with no recurrence (patients with no recurrence), and a group of the healthy subjects (healthy subjects). The patients with recurrence of urolithiasis (that is, the patients with early recurrence and the patients with late recurrence) were patients in whom formation of a new urinary calculus was detected by plain CT examination or plain X-ray examination after spontaneous stone passage, or stone passage or lithotripsy by treatment. Note that the patients with recurrence of urolithiasis excluded in advance patients in whom recurrence of urolithiasis due to a remaining urinary calculus was confirmed. Further, the patients with recurrence of urolithiasis excluded in advance patients in whom a urinary calculus was determined to be a cystine calculus, a uric acid calculus, or a magnesium ammonium phosphate calculus by calculus analysis. That is, the patients with recurrence of urolithiasis were patients in whom a urinary calculus was determined to be a calcium oxalate calculus or a calcium phosphate calculus by calculus analysis. Further, the patients with recurrence of urolithiasis excluded in advance patients diagnosed with renal tubular acidosis or hyperparathyroidism (that is, patients in a state likely to form a specific urinary calculus).

FIG. 20 shows the results of comparing the basic data for the four groups. The basic data is data including age, BMI, sex, the presence or absence of diabetes, the presence or absence of hyperlipidemia, the presence or absence of hypertension, the presence or absence of oral intake of citric acid, the presence or absence of a surgical history related to urolithiasis, and the presence or absence of a staghorn calculus. As shown in FIG. 20, there was no statistically significant difference in the basic data among the four groups.

### (2) 24-hour urine collection test

In the four groups, a 24-hour urine collection test was performed. Specifically, after 24-hour accumulated urine was collected from the subjects using URINMATE (SB-KAWASUMI LABORATORIES, INC.) which is a 24-hour urine proportional sampling device, various components such as electrolytes in the 24-hour accumulated urine were analyzed using an analyzer. Further, supersaturation indices (SI) of calcium oxalate (CaOx) and calcium phosphate (CaP) were calculated as SI.CaOx and SI.CaP using PHREEQC which is software for geochemical calculations. At this time, in the present example, among various CaOx and CaP, CaOx and CaP having the lowest solubility under a human body temperature condition were used to calculate the supersaturation indices. In detail, a supersaturation index of calcium oxalate monohydrate (COM) was used for SI.CaOx, and a supersaturation index of hydroxyapatite (HAP) was used for SI.CaP.

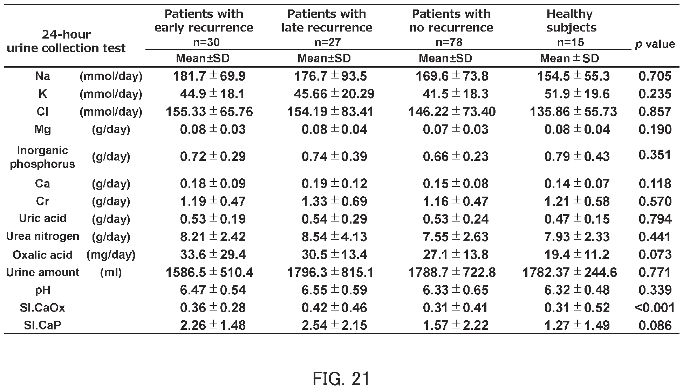

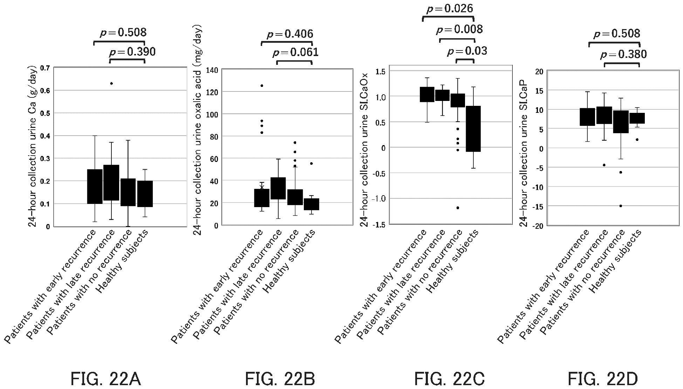

The results of the 24-hour urine collection test are shown in FIG. 21 and FIG. 22. In FIG. 21, the results of the 24-hour urine collection test are results including sodium (Na), potassium (K), chlorine (Cl), magnesium (Mg), inorganic phosphorus, calcium (Ca), creatinine (Cr), uric acid, urea nitrogen, oxalic acid, urine volume, pH, SI.CaOx, and SI.CaP. Further, in FIG. 22A, the Ca is shown as 24-hour accumulated urine Ca. In FIG. 22B, the oxalic acid is shown as 24-hour accumulated urine oxalic acid. In FIG. 22C, the SI.CaOx is shown as 24-hour accumulated urine SI.CaOx. In FIG. 22D, the SI.CaP is shown as 24-hour accumulated urine SI.CaP. As shown in FIG. 21 and FIG. 22, the SI.CaOx in each group of patients with urolithiasis was statistically significantly higher compared to the group of healthy subjects. In contrast, as shown in FIG. 21 and FIG. 22, there was no statistically significant difference in the SI.CaOx among the three groups of patients with urolithiasis (that is, the group of patients with early recurrence, the group of patients with late recurrence, and the group of patients with no recurrence). Further, as shown in FIG. 21 and FIG. 22, there was no statistically significant difference in other results of the 24-hour urine collection test among the four groups.

From the above results, it was found that SI.CaOx in the 24-hour urine collection test could differentiate the group of healthy subjects and the group of patients with urolithiasis. In contrast, it was found that the results of the 24-hour urine collection test could not necessarily differentiate the presence or absence of recurrence of urolithiasis and the timing of recurrence of urolithiasis among the groups of patients with urolithiasis.

### (3) Insolubilized material in cooled urine sample

The urine sample of the subject collected in the 24-hour urine collection test was processed in the same manner as in Example 1 (3) except that the sample was centrifuged under conditions of 3000 rpm for 10 minutes, to obtain a filtrate after cooling (that is, a cooled urine sample). The types of insolubilized materials in the cooled filtrate were classified by observation using an optical microscope and analysis by Raman spectroscopy.

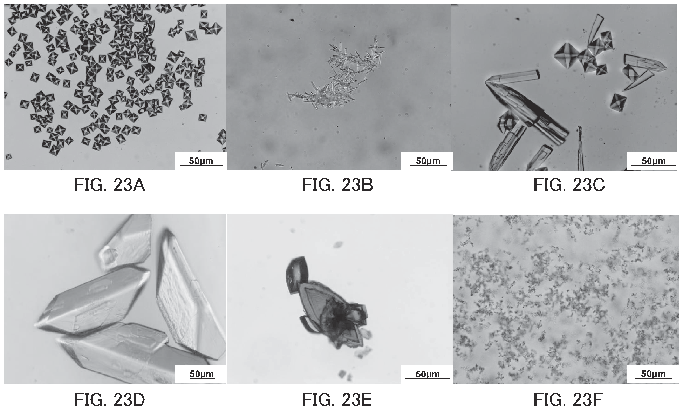

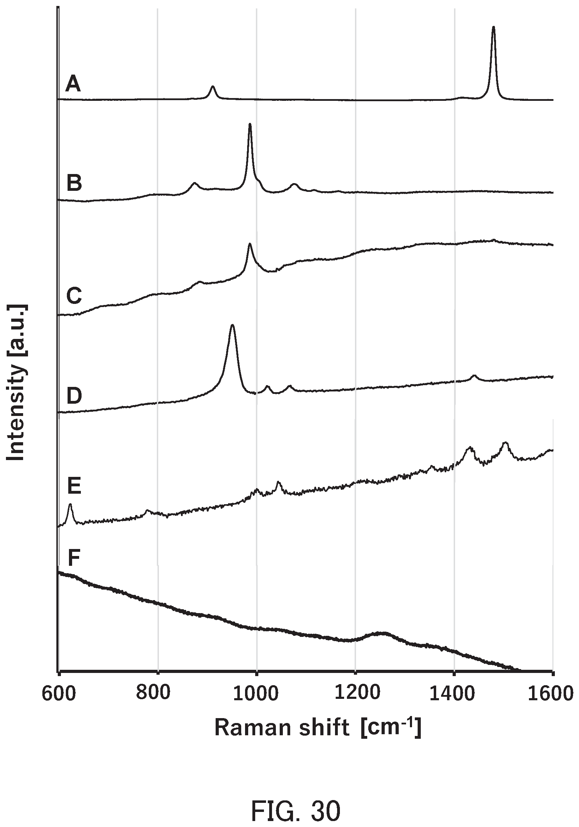

As shown in FIG. 23, by observation using an optical microscope, 6 types of insolubilized materials A to F exhibiting various shapes were detected in the cooled filtrate. Further, as shown in FIG. 30, as a result of analysis by Raman spectroscopy, among the insolubilized materials detected using the optical microscope, it was verified that the insolubilized material A exhibiting a regular octahedral shape was a crystal of calcium oxalate dihydrate (COD), the insolubilized materials B and C exhibiting a rosette-like or plate-like shape were crystals of calcium monohydrogen phosphate dihydrate (Brushite), the insolubilized material D exhibiting a bulk shape was a crystal of magnesium ammonium phosphate, the insolubilized material E exhibiting a rhombus shape was a crystal of uric acid, and the insolubilized material F was a crystal of carbonate apatite.

### (4) Examination of relationship between timing of recurrence of urolithiasis and insolubilized material

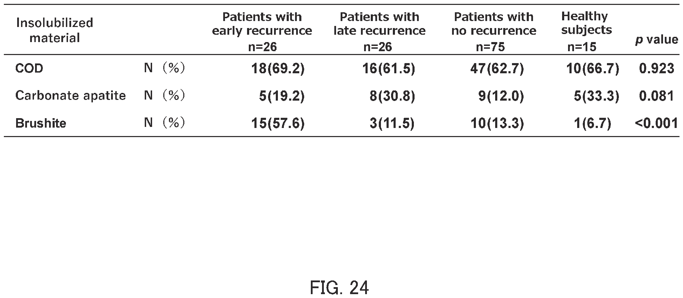

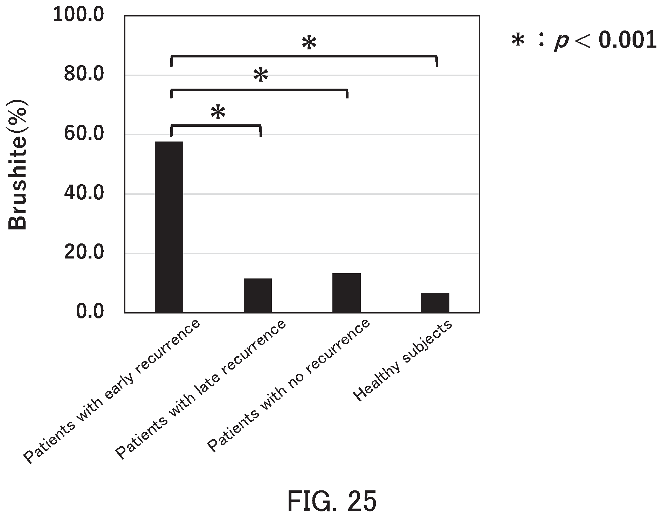

For the four groups, the relationship between the timing of recurrence of urolithiasis and an insolubilized material in a cooled urine sample was examined. Note that subjects in whom a crystal of magnesium ammonium phosphate or a crystal of uric acid was detected were excluded from the subjects of this examination. As shown in FIG. 24 and FIG. 25, the number of subjects in whom an insolubilized material of Brushite was detected was statistically significantly larger in the group of patients with early recurrence compared to other groups. In detail, the proportion of subjects in whom an insolubilized material of Brushite was detected was 57.6% in the patients with early recurrence, 11.5% in the patients with late recurrence, 13.3% in the patients with no recurrence, and 6.7% in the healthy subjects. In contrast, the proportion of subjects in whom an insolubilized material of COD was detected exceeded 60% in each of the groups. Further, the proportion of subjects in whom an insolubilized material of carbonate apatite was detected varied from 10% to 30% among the four groups. However, as shown in FIG. 24, there was no statistically significant difference in the number of subjects in whom an insolubilized material of COD or an insolubilized material of carbonate apatite was detected, among the four groups.

From the above results, it was found that a possibility that at least one of calcium phosphate crystals or amorphous calcium phosphate is detected in a cooled urine sample was high, in a patient with early recurrence of urolithiasis. In contrast, it was found that at least one of calcium oxalate crystals or amorphous calcium oxalate is universally detected in a cooled urine sample, regardless of the timing of recurrence of urolithiasis, the presence or absence of recurrence of urolithiasis, and the presence or absence of urolithiasis.

### (5) Evaluation of insolubilized material of calcium phosphate as biomarker for determining early recurrence of urolithiasis

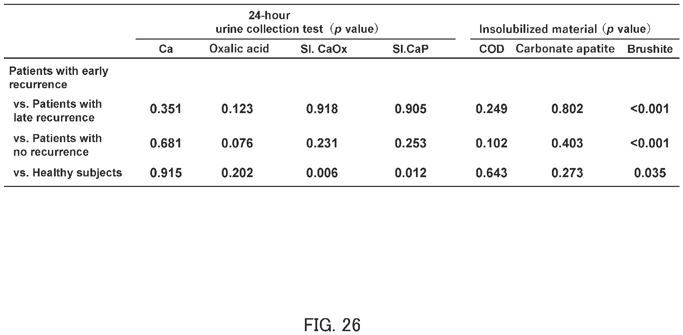

For the four groups, the relationship between the timing of recurrence of urolithiasis and an insolubilized material was further examined by logistic regression analysis. In the logistic regression analysis, the presence or absence of recurrence of urolithiasis was used as an objective variable, and results of the 24-hour urine collection test (Ca, oxalic acid, SI.CaOx, SI.CaP) and the presence or absence of an insolubilized material (COD, carbonate apatite, Brushite) were used as explanatory variables. As shown in FIG. 26, the presence or absence of an insolubilized material of Brushite was a factor that statistically significantly influenced the presence or absence of recurrence of urolithiasis in the group of patients with early recurrence, when compared to other groups (that is, the group of patients with late recurrence, the group of patients with no recurrence, and the group of healthy subjects). Further, SI.CaOx and SI.CaP were factors that statistically significantly influenced the presence or absence of recurrence of urolithiasis in the group of patients with early recurrence when compared to the group of healthy subjects. In contrast, other explanatory variables could not be verified to have a statistically significant influence on the presence or absence of recurrence of urolithiasis.

From the above results, it was found that at least one of calcium phosphate crystals or amorphous calcium phosphate can be used as a biomarker for determining early recurrence of urolithiasis. Further, it was found that at least one of calcium phosphate crystals or amorphous calcium phosphate can be used in a method for determining early recurrence of urolithiasis, which determines early recurrence of urolithiasis in a subject based on a detection result of at least one of calcium phosphate crystals or amorphous calcium phosphate.

### (6) Correlation between SI.COD and SI.Brushite in subjects in whom insolubilized material was observed in cooled urine sample

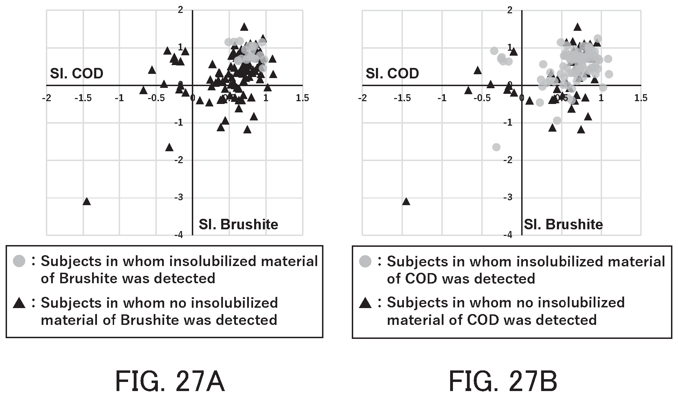

Supersaturation indices (SI) of calcium oxalate dihydrate (COD) and calcium monohydrogen phosphate dihydrate (Brushite) were calculated as SI.COD and SI.Brushite using PHREEQC, which is software for geochemical calculations. In the subjects in whom an insolubilized material of Brushite or an insolubilized material of COD was detected in the cooled urine sample, the correlation between SI.COD and SI.Brushite was examined for each type of insolubilized material. In FIG. 27A, the subjects in whom an insolubilized material of Brushite was detected are indicated by circle symbols, and the subjects in whom an insolubilized material of Brushite was not detected are indicated by triangle symbols. Further, in FIG. 27B, the subjects in whom an insolubilized material of COD was detected are indicated by circle symbols, and the subjects in whom an insolubilized material of COD was not detected are indicated by triangle symbols. As shown in FIG. 27A, a correlation between SI.COD and SI.Brushite was observed in the subjects in whom an insolubilized material of Brushite was detected. Further, as shown in FIG. 27B, a correlation between SI.COD and SI.Brushite was observed in the subjects in whom an insolubilized material of COD was detected.

### (7) Comparative examination of size and amount of insolubilized material of Brushite in patients with early recurrence of urolithiasis, patients with late recurrence of urolithiasis, and patients with no recurrence of urolithiasis

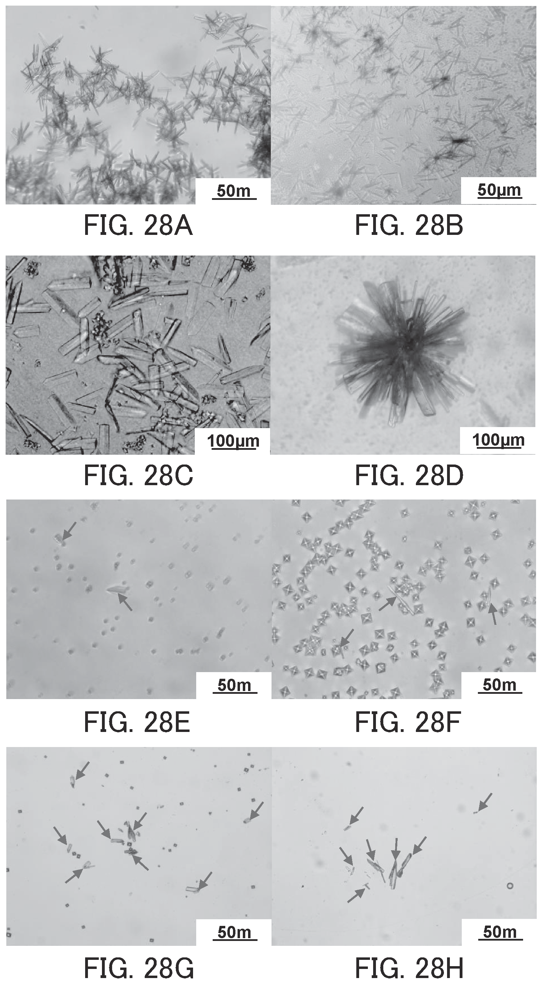

The size and amount of the insolubilized material of Brushite detected in the cooled urine sample were comparatively examined among the patients with early recurrence of urolithiasis, the patients with late recurrence of urolithiasis, and the patients with no recurrence of urolithiasis, by observation using an optical microscope. Photographs in FIGs. 28A to 28D show the insolubilized materials of Brushite detected in the cooled urine sample of the patients with early recurrence of urolithiasis. A photograph in FIG. 28E shows the insolubilized material of Brushite detected in the cooled urine sample of the patients with late recurrence of urolithiasis. Photographs in FIGs. 28F to 28H show the insolubilized materials of Brushite detected in the cooled urine sample of the patients with no recurrence of urolithiasis. Note that, in FIGs. 28A to 28D, the insolubilized materials of Brushite detected in the cooled urine sample are all the insolubilized materials included in the photographs. In contrast, in FIGs. 28E to 28H, the insolubilized materials of Brushite detected in the cooled urine sample are insolubilized materials indicated by the arrow symbols in the photographs. Comparing FIGs. 28A to 28B and FIGs. 28E to 28H, the amount of the insolubilized materials of Brushite detected in the cooled urine sample was remarkably larger in the patients with early recurrence of urolithiasis than in the patients with late recurrence of urolithiasis and the patients with no recurrence of urolithiasis. From this result, it can be said that the amount of at least one of calcium phosphate crystals or amorphous calcium phosphate in the patients with early recurrence of urolithiasis is, for example, about 100 or more per one visual field under a condition where a lens magnification is set to 20 times. Further, comparing FIGs. 28C to 28D and FIGs. 28E to 28H, the size of the insolubilized material of Brushite detected in the cooled urine sample was remarkably larger in the patients with early recurrence of urolithiasis than in the patients with late recurrence of urolithiasis and the patients with no recurrence of urolithiasis. From this result, it can be said that the size of at least one of calcium phosphate crystals or amorphous calcium phosphate in the patients with early recurrence of urolithiasis is, for example, about 100 µm or more.

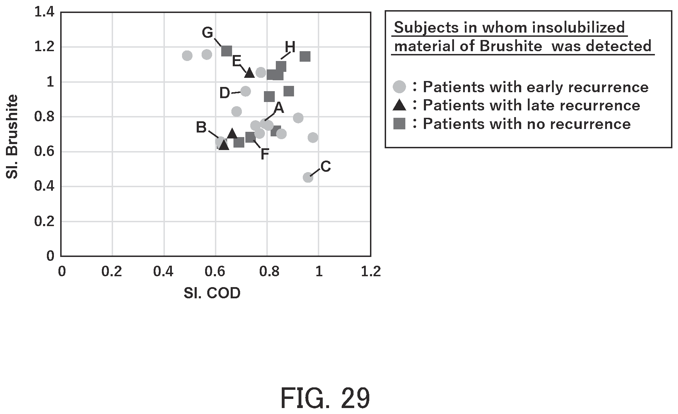

Further, the correlation between SI.COD and SI.Brushite in each group of patients with urolithiasis was examined. In FIG. 29, symbols A to D indicate patients with early recurrence in whom the insolubilized materials shown in FIGs. 28A to D were detected. In FIG. 29, symbol E indicates a patient with late recurrence in whom the insolubilized material shown in FIG. 28E was detected. In FIG. 29, symbols F to H indicate patients with no recurrence in whom the insolubilized materials shown in FIGs. 28F to H were detected. Note that, in FIG. 29, the patients with early recurrence are indicated by circle symbols, the patients with late recurrence are indicated by triangle symbols, and the patients with no recurrence are indicated by square symbols. As shown in FIG. 29, the correlation between SI.COD and SI.Brushite was not observed in the patients with early recurrence of urolithiasis, the patients with late recurrence of urolithiasis, and the patients with no recurrence of urolithiasis.

From the above results, it was found that the amount of at least one of calcium phosphate crystals or amorphous calcium phosphate was larger in a patient with early recurrence of urolithiasis, compared to a patient with late recurrence of urolithiasis. Further, it was found that the size of at least one of calcium phosphate crystals or amorphous calcium phosphate was larger in a patient with early recurrence of urolithiasis, compared to a patient with late recurrence of urolithiasis. Therefore, it was presumed that early recurrence of urolithiasis can be determined based on the size and amount of at least one of calcium phosphate crystals or amorphous calcium phosphate.

Therefore, it was presumed that the size and amount of at least one of calcium phosphate crystals or amorphous calcium phosphate can be used as a biomarker for determining early recurrence of urolithiasis. Further, it was presumed that the size and amount of at least one of calcium phosphate crystals or amorphous calcium phosphate can be used in a method for determining early recurrence of urolithiasis, which determines early recurrence of urolithiasis in a subject based on a detection result of the size and amount of at least one of calcium phosphate crystals or amorphous calcium phosphate.

Further, it was found that the size and amount of the insolubilized material of Brushite are not necessarily associated with SI.COD and SI.Brushite. Therefore, it was presumed that an evaluation based only on inorganic components such as SI.COD and SI.Brushite cannot necessarily determine early recurrence of urolithiasis. In contrast, an evaluation based on an insolubilized material in a cooled urine sample can grasp formation of a urinary calculus under a comprehensive environment including organic components such as proteins. Therefore, it was presumed that an evaluation based on at least one of calcium phosphate crystals or amorphous calcium phosphate in a cooled urine sample can determine early recurrence of urolithiasis.

While the present disclosure has been described above with reference to illustrative embodiments and examples, the present disclosure is by no means limited thereto. Various changes and modifications that may become apparent to those skilled in the art may be made in the configuration and specifics of the present disclosure without departing from the scope of the present disclosure. Further, each embodiment may be combined with other embodiments as appropriate.

This application claims priority from Japanese Patent Application No. 2024-004939 filed on January 17, 2024, and Japanese Patent Application No. 2024-129618 filed on August 6, 2024. The entire subject matter of the Japanese Patent Application is incorporated herein by reference.

### SUPPLEMENTARY NOTE

The whole or part of the example embodiments disclosed above can be described as, but not limited to, the following supplementary notes.

### Biomarker

(Supplementary Note 1) A biomarker for determining development of urolithiasis, including at least one of calcium phosphate crystals or amorphous calcium phosphate in urine.
(Supplementary Note 2) The biomarker according to supplementary note 1, wherein the calcium phosphate includes calcium monohydrogen phosphate.
(Supplementary Note 3) The biomarker according to supplementary note 1 or 2, wherein the calcium phosphate includes calcium monohydrogen phosphate dihydrate.
(Supplementary Note 4) The biomarker according to any one of supplementary notes 1 to 3, wherein the urine is accumulated urine.
(Supplementary Note 5) The biomarker according to any one of supplementary notes 1 to 4, wherein the urine is 24-hour accumulated urine.
(Supplementary Note 6) The biomarker according to any one of supplementary notes 1 to 5, wherein the biomarker is a biomarker for determining recurrence of urolithiasis.
(Supplementary Note 7) The biomarker according to any one of supplementary notes 1 to 6, wherein the biomarker is a biomarker for determining early recurrence of urolithiasis.

### Determination method

(Supplementary Note 8) A method for determining development of urolithiasis, including:
   detecting the biomarker according to any one of supplementary notes 1 to 5 in urine from a subject, and
   determining development of urolithiasis in the subject based on a result of the detection of the biomarker.
(Supplementary Note 9) The method according to supplementary note 8, further including performing insolubilization processing on the urine from the subject to form the biomarker.
(Supplementary Note 10) The method according to supplementary note 9, wherein
   in the performing insolubilization processing, the insolubilization processing is supersaturation.
(Supplementary Note 11) The method according to supplementary note 10, wherein the supersaturation includes cooling.
(Supplementary Note 12) The method according to supplementary note 11, wherein a temperature of the cooling is 0 to 4°C.
(Supplementary Note 13) The method according to supplementary note 11, wherein a time of the cooling is 24 hours or more.
(Supplementary Note 14) The method according to supplementary note 11, wherein a temperature of the cooling is 0 to 4°C, and a time of the cooling is 24 hours or more.
(Supplementary Note 15) The method according to any one of supplementary notes 8 to 14, wherein
   when the biomarker in the urine from the subject is detected in the detecting, the determining includes determining that the subject has developed urolithiasis.
(Supplementary Note 16) The method according to any one of supplementary notes 8 to 15, wherein recurrence of urolithiasis in the subject is determined in the determining.
(Supplementary Note 17) The method according to any one of supplementary notes 8 to 16, wherein early recurrence of urolithiasis in the subject is determined in the determining.

### Screening method

(Supplementary Note 18) A method for screening for a candidate substance for suppressing urolithiasis, including selecting a formation-inhibiting substance that inhibits formation of at least one of calcium phosphate crystals or amorphous calcium phosphate from test substances, as the candidate substance for suppressing urolithiasis.
(Supplementary Note 19) The method according to supplementary note 18, wherein the calcium phosphate includes calcium monohydrogen phosphate.
(Supplementary Note 20) The method according to supplementary note 18 or 19, wherein the calcium phosphate includes calcium monohydrogen phosphate dihydrate.
(Supplementary Note 21) The method according to any one of supplementary notes 18 to 20, further including:
   performing insolubilization processing in the presence of the test substances to form at least one of the calcium phosphate crystals or the amorphous calcium phosphate, and
   detecting at least one of the calcium phosphate crystals or the amorphous calcium phosphate,
   wherein
   in the selecting, the candidate substance for suppressing urolithiasis is selected from the test substances based on a result of the detection of at least one of the calcium phosphate crystals or the amorphous calcium phosphate.
(Supplementary Note 22) The method according to supplementary note 21, wherein
   in the performing insolubilization processing, the insolubilization processing is supersaturation.
(Supplementary Note 23) The method according to supplementary note 22, wherein the supersaturation includes cooling.
(Supplementary Note 24) The method according to supplementary note 23, wherein a temperature of the cooling is 0 to 4°C.
(Supplementary Note 25) The method according to supplementary note 23, wherein a time of the cooling is 24 hours or more.
(Supplementary Note 26) The method according to supplementary note 23, wherein a temperature of the cooling is 0 to 4°C, and a time of the cooling is 24 hours or more.
(Supplementary Note 27) The method according to any one of supplementary notes 18 to 26, wherein
   in the selecting, a test substance for which the amount of at least one of the calcium phosphate crystals or the amorphous calcium phosphate is lower than that in a control in the absence of the test substance is selected as the candidate substance for suppressing urolithiasis.

### Use

(Supplementary Note 28) Use of at least one of calcium phosphate crystals or amorphous calcium phosphate in urine for determining development of urolithiasis.
(Supplementary Note 29) The use according to supplementary note 28, wherein the calcium phosphate includes calcium monohydrogen phosphate.
(Supplementary Note 30) The use according to supplementary note 28 or 29, wherein the calcium phosphate includes calcium monohydrogen phosphate dihydrate.
(Supplementary Note 31) The use according to any one of supplementary notes 28 to 30, wherein the urine is accumulated urine.
(Supplementary Note 32) The use according to any one of supplementary notes 28 to 31, wherein the urine is 24-hour accumulated urine.
(Supplementary Note 33) The use according to any one of supplementary notes 28 to 32, wherein the development of urolithiasis is recurrence of urolithiasis.
(Supplementary Note 34) The use according to any one of supplementary notes 28 to 33, wherein the development of urolithiasis is early recurrence of urolithiasis.

### Industrial Applicability

The present disclosure allows, for example, development of urolithiasis to be accurately determined. Therefore, the present disclosure is applicable to various fields including the medical field.

## Claims

1. A biomarker for determining development of urolithiasis, comprising at least one of calcium phosphate crystals or amorphous calcium phosphate in urine.

2. The biomarker according to claim 1, wherein the biomarker is a biomarker for determining recurrence of urolithiasis.

3. A method for determining development of urolithiasis, comprising:
detecting the biomarker according to claim 1 in urine from a subject, and
determining development of urolithiasis in the subject based on a result of the detection of the biomarker.

4. The method according to claim 3, wherein
recurrence of urolithiasis in the subject is determined in the determining.

5. The method according to claim 3, further comprising performing insolubilization processing on the urine from the subject to form the biomarker.

6. The method according to claim 5, wherein
in the performing insolubilization processing, the insolubilization processing is supersaturation.

7. The method according to claim 3, wherein
when the biomarker in the urine from the subject is detected in the detecting,
the determining includes determining that the subject has developed urolithiasis.

8. A method for screening for a candidate substance for suppressing urolithiasis, comprising selecting a formation-inhibiting substance that inhibits formation of at least one of calcium phosphate crystals or amorphous calcium phosphate from test substances, as the candidate substance for suppressing urolithiasis.

9. The method according to claim 8, further comprising:
performing insolubilization processing in the presence of the test substances to form at least one of the calcium phosphate crystals or the amorphous calcium phosphate, and
detecting at least one of the calcium phosphate crystals or the amorphous calcium phosphate, wherein
in the selecting, the candidate substance for suppressing urolithiasis is selected from the test substances based on a result of the detection of at least one of the calcium phosphate crystals or the amorphous calcium phosphate.

10. The method according to claim 9, wherein
in the selecting, a test substance for which the amount of at least one of the calcium phosphate crystals or the amorphous calcium phosphate is lower than that in a control in the absence of the test substance is selected as the candidate substance for suppressing urolithiasis.

11. The method according to claim 9 or 10, wherein, in the performing insolubilization processing, the insolubilization processing is supersaturation.
